# EUROPEAN PATENT APPLICATION

(11) **EP 4 711 362 A1**
(43) Date of publication of application: **18.03.2026**
(21) Application number: 24803008.2
(22) Date of filing: 08.05.2024
(51) Int. Cl.: C07D 401/14, A61K 31/4709, A61P 35/00

(54) **LIGAND TARGETING FIBROBLAST ACTIVATION PROTEIN**

(30) Priority: 08.05.2023 CN 202310510526; 13.03.2024 CN 202410286316
(71) Applicant: Jiangsu Hengrui Pharmaceuticals Co., Ltd., Lianyungang, Jiangsu 222047 (CN); Tianjin Hengrui Medicine Co., Ltd., Tianjin 300303 (CN)
(72) Inventor: ZHOU, Shunguang, Tianjin 300303 (CN); GE, Xinyue, Tianjin 300303 (CN); SUN, Jiyun, Tianjin 300303 (CN); LI, Zhongqing, Tianjin 300303 (CN); SONG, Zihui, Tianjin 300303 (CN); ZHANG, Mengyi, Tianjin 300303 (CN); GUO, Feihu, Tianjin 300303 (CN)
(74) Representative: Potter Clarkson
(86) International application number: PCT/CN2024/091688
(87) International publication number: WO 2024/230728

(57) **Abstract**

The present disclosure relates to a ligand targeting a fibroblast activation protein, and particularly to a ligand represented by formula (I) and targeting a fibroblast activation protein, and a radioactive label thereof, wherein each substituent is defined in the description. The ligand can be used for imaging diseases or disorders related to the fibroblast activation protein, or treating the diseases or disorders related to the fibroblast activation protein.

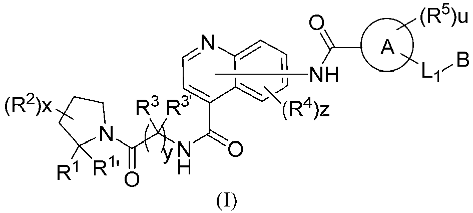

## Description

### Technical Field

The present disclosure relates to a ligand targeting fibroblast activation protein and a radioactive label thereof, and the pharmaceutical use.

### Background Art

Fibroblast activation protein (FAP) is a membrane-bound gelatinase that can promote tumour growth and progression, and is overexpressed in cancer-associated fibroblasts. FAP, due to its low expression in normal organs, represents an ideal target for the development of targeted small molecule drug conjugates (SMDCs) and small molecule radioconjugates (SMRCs).

Studies on fibroblast activation protein (FAP) inhibitors include WO 2019154886 A, WO 2019154859 A, WO 2019118932 A, WO 2019083990 A, WO 2013107820 A, WO 2018111989 A, WO 2023/057457 A, etc. However, developing effective FAP-binding agents with excellent targeting properties, as well as targeted small molecule drug conjugates and small molecule radioconjugates based thereon, remains a challenging task. It is particularly desirable that small molecule radioconjugates exhibit favourable tumour-to-organ biodistribution, high activity labelling, and/or good compound stability.

### Summary of the Invention

The present disclosure relates to a ligand for fibroblast activation protein (FAP) for active delivery of a radioactive payload to a disease site.

The present disclosure provides a compound represented by formula (I) or a pharmaceutically acceptable salt thereof, 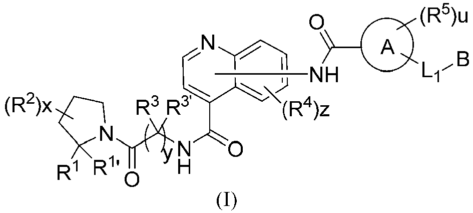
wherein R¹ and R¹' are each independently selected from hydrogen, cyano, carboxyl, sulfo, phosphono or B(OH)₂;
each R² is independently selected from hydroxyl, halogen, C₁₋₆ alkyl, halo-C₁₋₆ alkyl, -NR'R", -O-C₁₋₆ alkyl or -S-C₁₋₆ alkyl;
R³ and R³' are each independently selected from hydrogen, hydroxyl, halogen or C₁₋₆ alkyl;
R⁴ is selected from hydroxyl, halogen, C₁₋₆ alkyl, halo-C₁₋₆ alkyl, -NR'R", -O-C₁₋₆ alkyl or -S-C₁₋₆ alkyl;
ring A is selected from 3- to 12-membered cycloalkylene or 3- to 12-membered heterocycloalkylene;
R⁵ is selected from halogen, hydroxyl, C₁₋₆ alkyl, halo-C₁₋₆ alkyl, -NR'R" or - O-C₁₋₆ alkyl;
L₁ is selected from a linking bond, -NH-, -CH₂-NH- or -CH₂CH₂-NH-;
x is selected from 0, 1, 2 or 3;
R' and R" are each independently selected from hydrogen, C₁₋₆ alkyl or halo-C₁₋₆ alkyl;
y is selected from 1 or 2;
z is selected from 0, 1, 2 or 3;
u is selected from 0, 1 or 2; and
B is selected from any optically- or radio-labelled functional group suitable for optical imaging, positron emission tomography, single-photon emission computed tomography or radiation therapy.

In an alternative embodiment, B is composed of a chelating agent and a radioactive element.

In an alternative embodiment, ring A is selected from 4- to 7-membered cycloalkylene, e.g., cyclobutylene, cyclopentylene, cyclohexylene or cycloheptylene.

In an alternative embodiment, ring A is cyclohexylene.

In an alternative embodiment, R¹ and R¹' are each independently selected from hydrogen or cyano.

In an alternative embodiment, each R² is independently selected from hydroxyl, halogen or C₁₋₆ alkyl, and x is selected from 2 or 3.

In an alternative embodiment, each R² is independently selected from fluorine or chlorine, and x is selected from 2 or 3.

In an alternative embodiment, R³ and R³' are each independently selected from hydrogen, halogen or C₁₋₆ alkyl.

In an alternative embodiment, R³ and R³' are both hydrogen.

In an alternative embodiment, the compound represented by formula (I) or the pharmaceutically acceptable salt thereof provided by the present disclosure is a compound represented by formula (11-1) or a pharmaceutically acceptable salt thereof, 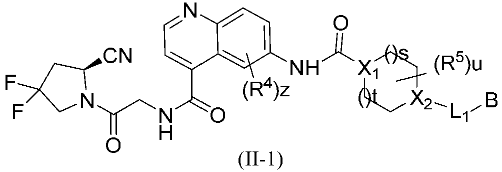
wherein X₁ and X₂ are each independently selected from CH or N,
s and t are each independently selected from 0, 1 or 2, and
R⁴, R⁵, L₁, B, z and u are as defined in formula (I).

In an alternative embodiment, X₁ is CH, and X₂ is N.

In an alternative embodiment, X₁ is N, and X₂ is CH.

In an alternative embodiment, X₁ is CH, and X₂ is CH.

In an alternative embodiment, s and t are each independently selected from 0 or 1.

In an alternative embodiment, s and t are both 1.

In an alternative embodiment, L₁ is selected from a linking bond or -NH-.

In an alternative embodiment, L₁ is selected from CH₂-NH- or -CH₂CH₂-NH-.

In an alternative embodiment, L₁ is CH₂-NH-.

In an alternative embodiment, L₁ is -CH₂CH₂-NH-.

In an alternative embodiment, L₁ is CH, and X₂ is CH; s and t are both 1; L₁ is selected from CH₂-NH- or -CH₂CH₂-NH-.

In an alternative embodiment, X₁ is CH, and X₂ is CH; s and t are both 1; L₁ is -CH₂-NH-.

In an alternative embodiment, R⁴ is selected from hydroxyl, halogen or C₁₋₆ alkyl, and z is selected from 0, 1, 2 or 3.

In an alternative embodiment, R⁴ is selected from fluorine, chlorine, methyl or ethyl, and z is selected from 0 or 1.

In an alternative embodiment, R⁵ is selected from hydroxyl, halogen or C₁₋₆ alkyl, and u is selected from 0, 1 or 2.

In an alternative embodiment, R⁵ is selected from fluorine, chlorine, methyl or ethyl, and u is selected from 0 or 1.

In an alternative embodiment, R⁴ and R⁵ are each independently selected from fluorine, chlorine, methyl or ethyl, and z and u are each independently selected from 0 or 1.

In an alternative embodiment, the compound represented by formula (I) or the pharmaceutically acceptable salt thereof is a compound represented by formula (II-2) or a pharmaceutically acceptable salt thereof, 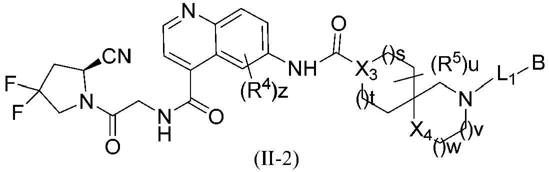
wherein X₃ and X₄ are each independently selected from CH, N or O;
s and t are each independently selected from 0, 1 or 2;
w and v are each independently selected from 0, 1 or 2; and
R⁴, R⁵, L₁, B, z and u are as defined in formula (I).

In an alternative embodiment, X₃ is CH, and X₄ is O.

In an alternative embodiment, X₃ is CH, and X₄ is N.

In an alternative embodiment, X₃ is N, and X₄ is CH.

In an alternative embodiment, s and t are each independently selected from 0 or 1, and w and v are each independently selected from 0 or 1.

In an alternative embodiment, s and t are both 1, and w and v are both 1.

In an alternative embodiment, L₁ is a linking bond.

In an alternative embodiment, L₁ is NH-.

In an alternative embodiment, L₁ is selected from CH₂-NH- or -CH₂CH₂-NH-.

In an alternative embodiment, L₁ is CH₂-NH-.

In an alternative embodiment, L₁ is -CH₂CH₂-NH-.

In an alternative embodiment, X₃ is CH, and X₄ is O; s and t are both 1, and w and v are both 1; L₁ is a linking bond.

In an alternative embodiment, R⁴ is selected from hydroxyl, halogen or C₁₋₆ alkyl, and z is selected from 0, 1, 2 or 3.

In an alternative embodiment, R⁴ is selected from fluorine, chlorine, methyl or ethyl, and z is selected from 0 or 1.

In an alternative embodiment, R⁵ is selected from hydroxyl, halogen or C₁₋₆ alkyl, and u is selected from 0, 1 or 2.

In an alternative embodiment, R⁵ is selected from fluorine, chlorine, methyl or ethyl, and u is selected from 0 or 1.

In an alternative embodiment, R⁴ and R⁵ are each independently selected from fluorine, chlorine, methyl or ethyl, and z and u are each independently selected from 0 or 1.

In an alternative embodiment, the chelating agent in B is selected from:

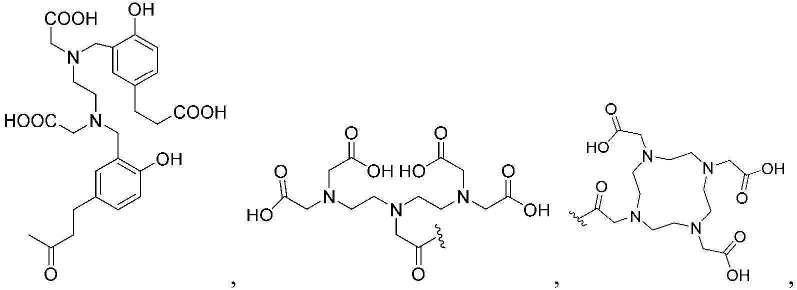

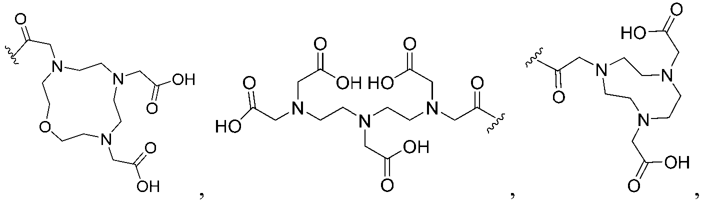

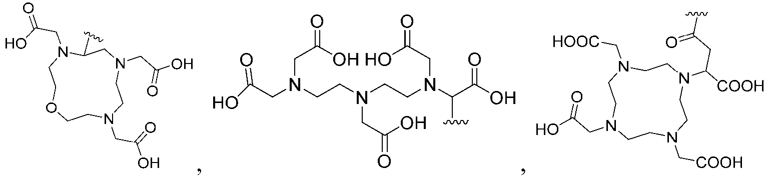

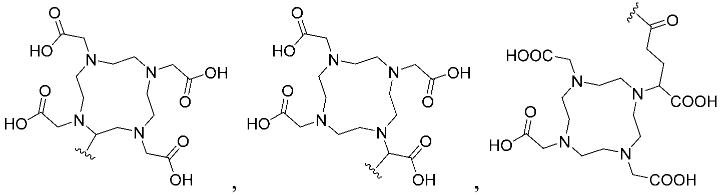

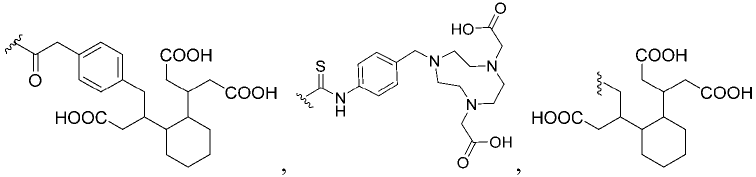

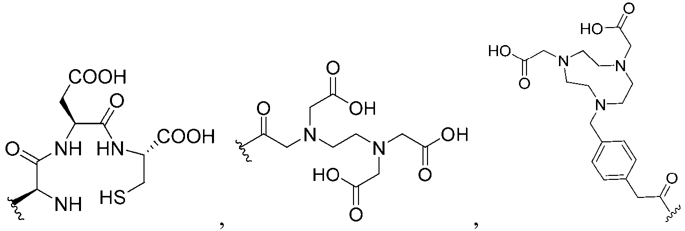

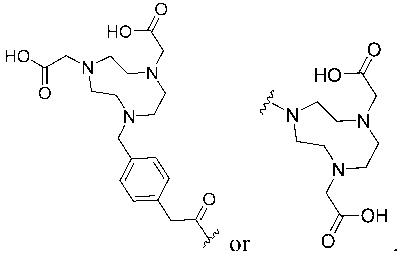

In an alternative embodiment, the chelating agent in B is

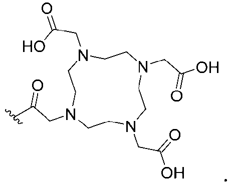

In an alternative embodiment, the chelating agent in B is

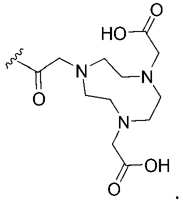

In an alternative embodiment, the radioactive element in B is selected from ²²³Ra, ⁸⁹Sr, ^{94m}Tc, ^{99m}Tc, ¹⁸⁶Re, ¹⁸⁸Re, ²⁰³Pb, ²¹²Pb, ⁶⁷Ga, ⁶⁸Ga, ⁴⁷Sc, ¹¹¹In, ⁹⁷Ru, ⁶²Cu, ⁶⁴Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ¹²³I, ¹²⁴I, ¹²⁵I, ¹⁸F, ²¹¹At, ²²⁵Ac, ⁸⁹Sr, ^{117m}Sn or ¹⁶⁹Er.

In an alternative embodiment, the radioactive element in B is ⁶⁴Cu.

In an alternative embodiment, the radioactive element in B is ⁶⁸Ga.

In an alternative embodiment, the radioactive element in B is ¹⁸F.

In an alternative embodiment, the radioactive element in B is selected from: ⁴³Sc, ⁴⁴Sc, ⁵¹Mn, ⁵²Mn, ¹⁵²Tb, ¹⁵⁵Tb, ²⁰¹TI, ⁷⁶Br, ⁷⁷Br, ⁸⁹Zr , ⁴⁷Sc, ⁶⁷Cu, ¹⁴⁹Tb, ²¹³Bi, ²²⁶Th, ²²⁷Th or ¹³¹I.

In an alternative embodiment, the present disclosure provides a compound as follows or a pharmaceutically acceptable salt thereof, 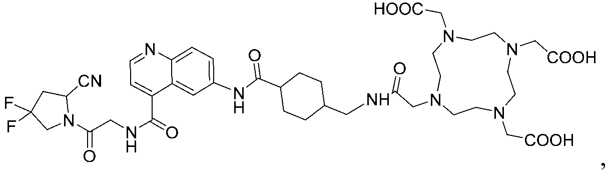 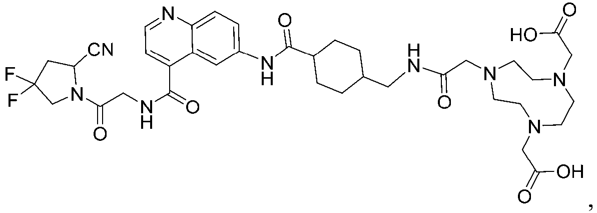 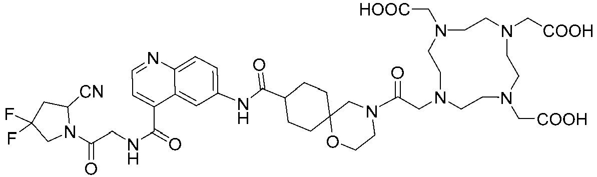 or

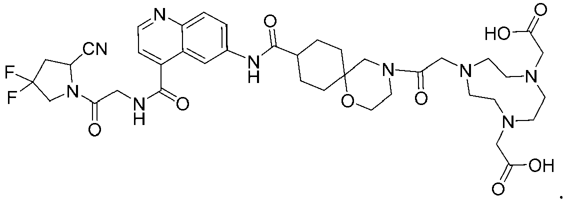

In an alternative embodiment, the present disclosure provides a compound as follows or a pharmaceutically acceptable salt thereof, 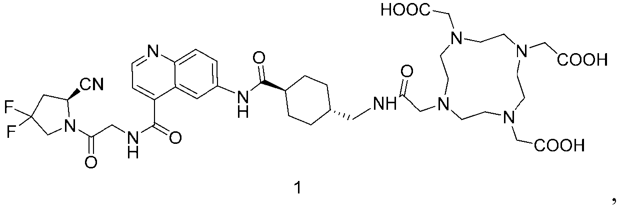 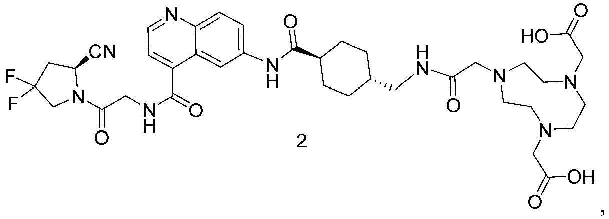 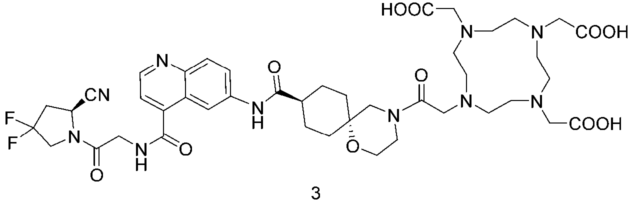 or

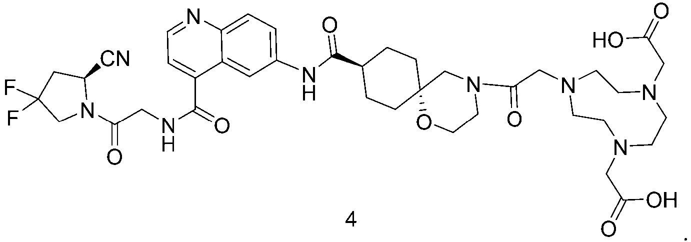

In an alternative embodiment, the present disclosure provides a compound as follows or a pharmaceutically acceptable salt thereof, 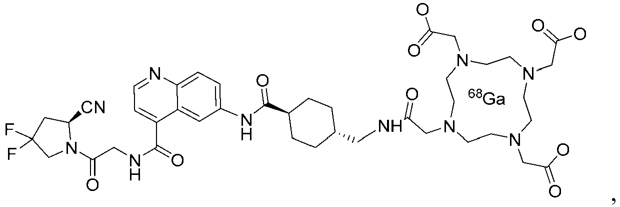 wherein the structural formula may also be

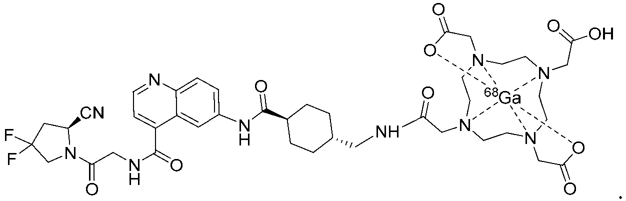

In an alternative embodiment, the present disclosure provides a compound as follows or a pharmaceutically acceptable salt thereof, 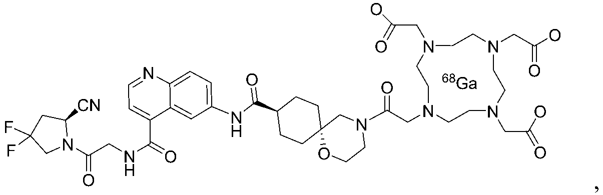 wherein the structural formula may also be

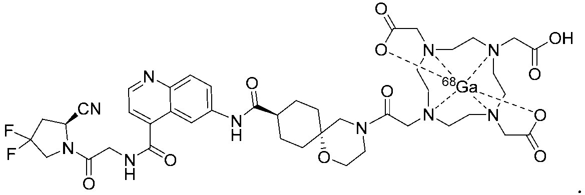

In an alternative embodiment, the present disclosure provides a compound as follows or a pharmaceutically acceptable salt thereof, 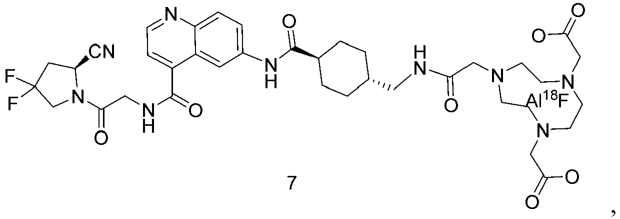 wherein the structural formula may also be

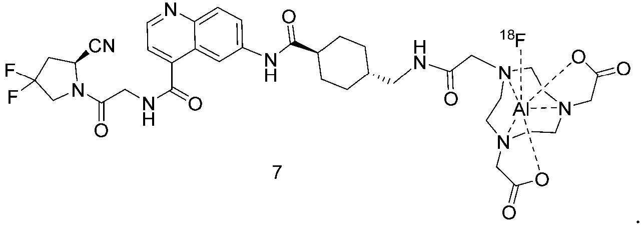

In an alternative embodiment, the present disclosure provides a compound as follows or a pharmaceutically acceptable salt thereof, 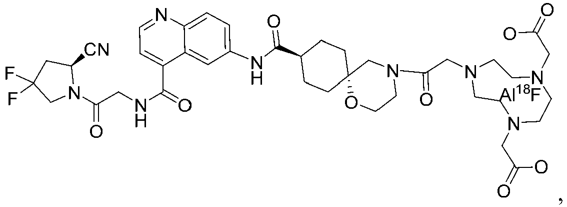 wherein the structural formula may also be

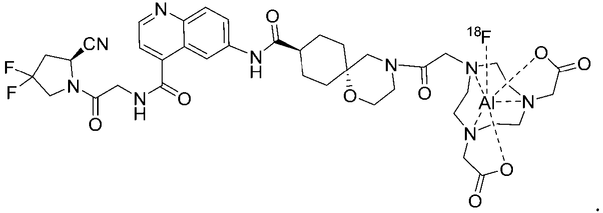

In an alternative embodiment, the present disclosure provides a compound as follows or a pharmaceutically acceptable salt thereof, 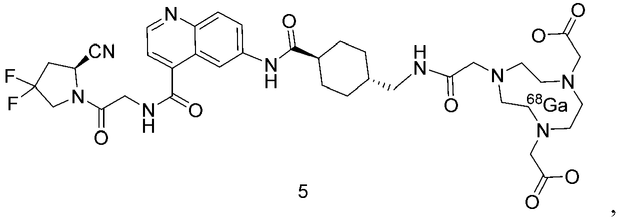 wherein the structural formula may also be

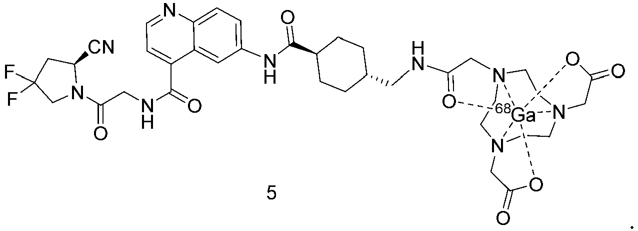

In an alternative embodiment, the present disclosure provides a compound as follows or a pharmaceutically acceptable salt thereof, 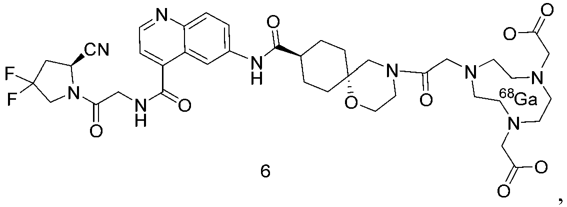 wherein the structural formula may also be

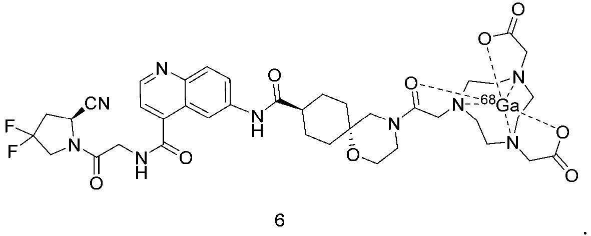

In an alternative embodiment, the present disclosure provides a compound as follows or a pharmaceutically acceptable salt thereof,

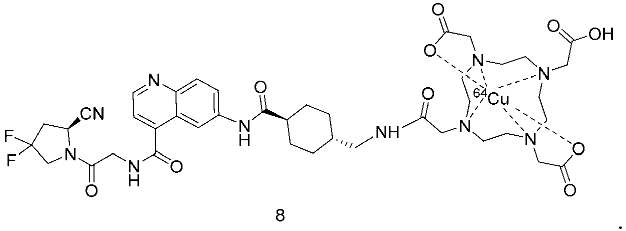

In an alternative embodiment, the present disclosure provides a compound as follows or a pharmaceutically acceptable salt thereof,

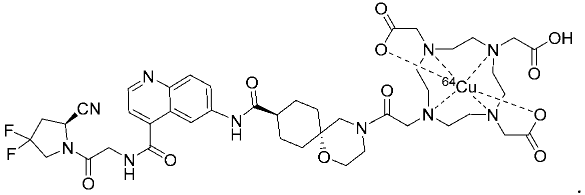

In an alternative embodiment, the present disclosure provides a compound as follows or a pharmaceutically acceptable salt thereof,

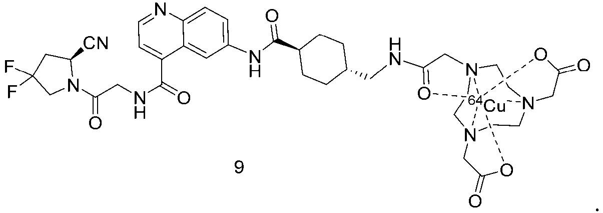

In an alternative embodiment, the present disclosure provides a compound as follows or a pharmaceutically acceptable salt thereof,

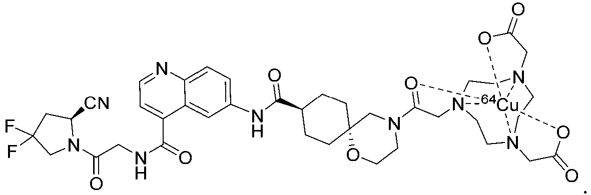

The present disclosure further provides a pharmaceutical composition comprising the aforementioned compound or a pharmaceutically acceptable salt thereof, and one or more pharmaceutically acceptable carriers, diluents or excipients.

In some embodiments, the unit dose of the pharmaceutical composition is 0.001 mg to 1000 mg.

In certain embodiments, the pharmaceutical composition comprises 0.01%-99.99% of the aforementioned compound or a pharmaceutically acceptable salt thereof, or an isotopic substitute thereof, on the basis of the total weight of the composition. In certain embodiments, the pharmaceutical composition comprises 0.1 %-99.9% of the aforementioned compound or a pharmaceutically acceptable salt thereof, or an isotopic substitute thereof. In certain embodiments, the pharmaceutical composition comprises 0.5%-99.5% of the aforementioned compound or a pharmaceutically acceptable salt thereof, or an isotopic substitute thereof. In certain embodiments, the pharmaceutical composition comprises 1%-99% of the aforementioned compound or a pharmaceutically acceptable salt thereof, or an isotopic substitute thereof. In certain embodiments, the pharmaceutical composition comprises 2%-98% of the aforementioned compound or a pharmaceutically acceptable salt thereof, or an isotopic substitute thereof.

In certain embodiments, the pharmaceutical composition comprises 0.01%-99.99% of the pharmaceutically acceptable excipient, on the basis of the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

In another aspect, the present disclosure discloses a method for preparing the aforementioned compound or a pharmaceutically acceptable salt thereof, or the pharmaceutical combination, the method comprising a step of complexing the compound represented by formula (I), formula (II-1) or formula (II-2) or the pharmaceutically acceptable salt thereof with a radioactive element.

The present disclosure further discloses a method for imaging a disease or disorder related to fibroblast activation protein, the method comprising steps of: 1) administering to a patient the aforementioned compound or a pharmaceutically acceptable salt thereof, or the aforementioned pharmaceutical composition; and 2) acquiring an image.

The present disclosure further discloses a method for inhibiting fibroblast activation protein, the method comprising administering to a patient the aforementioned compound or a pharmaceutically acceptable salt thereof, or the aforementioned pharmaceutical composition.

The present disclosure further discloses a method for diagnosing or treating a disease or disorder related to fibroblast activation protein, the method comprising administering to a patient the aforementioned compound or a pharmaceutically acceptable salt thereof, or the aforementioned pharmaceutical composition.

In an alternative embodiment, in the method for imaging a disease or disorder related to fibroblast activation protein, the method for inhibiting fibroblast activation protein, or the method for treating a disease or disorder related to fibroblast activation protein, an effective amount of the compound represented by formula (I), formula (II-1) or formula (II-2) or a pharmaceutically acceptable salt thereof, or the pharmaceutical composition comprising same is administered to a patient.

The present disclosure further discloses the use of the aforementioned compound or a pharmaceutically acceptable salt thereof, or the pharmaceutical composition in the preparation of a medicament for imaging a disease or disorder related to fibroblast activation protein, or for treating a disease or disorder related to fibroblast activation protein.

The present disclosure further relates to the compound represented by formula (I), formula (II-1) or formula (II-2) or a pharmaceutically acceptable salt thereof, or the pharmaceutical composition comprising same, for use as a medicament.

The present disclosure further relates to the compound represented by formula (I), formula (II-1) or formula (II-2) or a pharmaceutically acceptable salt thereof, or the pharmaceutical composition comprising same, for use in imaging a disease or disorder related to fibroblast activation protein.

The present disclosure further relates to the compound represented by formula (I), formula (II-1) or formula (II-2) or a pharmaceutically acceptable salt thereof, or the pharmaceutical composition comprising same, for use in inhibiting fibroblast activation protein.

The present disclosure further relates to the compound represented by formula (I), formula (II-1) or formula (II-2) or a pharmaceutically acceptable salt thereof, or the pharmaceutical composition comprising same, for use in diagnosing or treating a disease or disorder related to fibroblast activation protein.

The disease or disorder related to fibroblast activation protein described in the present disclosure is selected from proliferative diseases, chronic inflammation, fibrosis (liver, kidney, lung), tissue remodelling, scarring disorders, tissue infections or inflammatory lesions.

The present disclosure further discloses the use of the aforementioned compound or a pharmaceutically acceptable salt thereof, or the pharmaceutical composition in the preparation of a medicament for preventing, diagnosing, or treating proliferative diseases, chronic inflammation, fibrosis (liver, kidney, lung), tissue remodelling, scarring disorders, tissue infections or inflammatory lesions.

The proliferative diseases described in the present disclosure are selected from the group consisting of breast cancer, colorectal cancer, ovarian cancer, prostate cancer, pancreatic cancer, thyroid cancer, lung adenocarcinoma, kidney cancer, liver cancer, lung cancer, oesophageal cancer, hepatobiliary tract cancer, gastric cancer, nasopharyngeal cancer, head and neck cancer, bladder cancer, glioblastoma, peritoneal metastatic cancer, melanoma, fibrosarcoma, bone and connective tissue sarcoma, renal cell carcinoma, giant cell carcinoma, squamous cell carcinoma and adenocarcinoma, and benign tumours.

The chronic inflammation described in the present disclosure is selected from rheumatoid arthritis, osteoarthritis, Crohn's disease and atherosclerotic plaques.

The tissue remodelling described in the present disclosure occurs after myocardial infarction. The scarring disorders described in the present disclosure are selected from scar formation, scar tumours or scar keloids.

Provided is a method for preparing compound 7 as shown below, 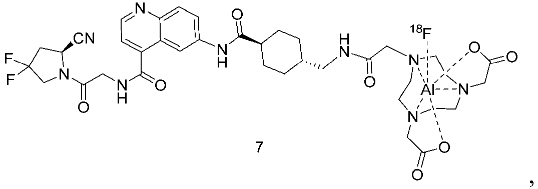 comprising a step of subjecting compound 2 to a reaction with ¹⁸F⁻ and AlCl₃ under acidic conditions (e.g., acetic acid/sodium acetate buffer) at high temperature (e.g., 75°C-100°C),

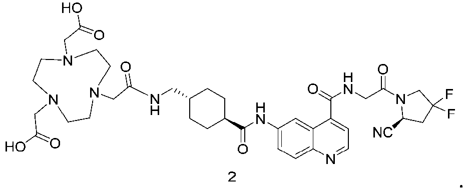

Provided is method for preparing compound 5 as shown below, 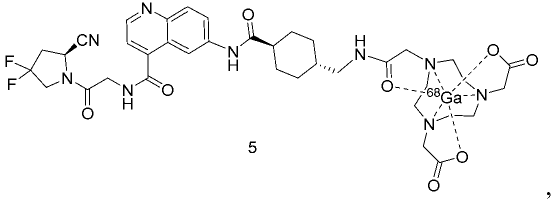 comprising a step of subjecting compound 2 to a reaction with ⁶⁸GaCl₃ under acidic conditions (e.g., acetic acid/sodium acetate buffer) at high temperature (e.g., 75°C-100°C).

Provided is a method for preparing compound 9 as shown below, 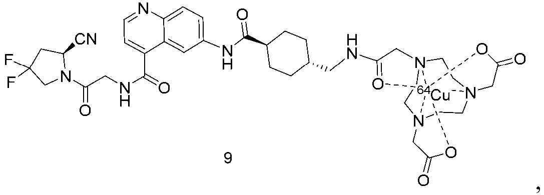 comprising a step of subjecting compound 2 to a reaction with ⁶⁴CuCl₂ under acidic conditions (e.g., acetic acid/sodium acetate buffer) at high temperature (e.g., 75°C-100°C).

Provided is a method for preparing compound 2, comprising a step of removing a tert-butyl protecting group from compound 2-a under acidic conditions (e.g., trifluoroacetic acid),

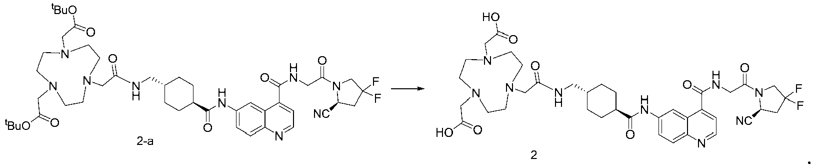

In an alternative embodiment, the aforementioned methods for preparing compounds 5, 7 and 9 each comprise a step of removing a tert-butyl protecting group from compound 2-a under acidic conditions (e.g., trifluoroacetic acid) to give compound 2,

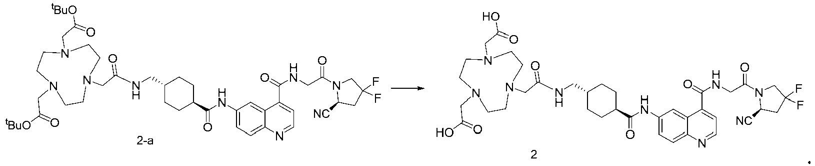

In an alternative embodiment, the aforementioned methods for preparing compounds 5, 7 and 9 or compound 2 each comprise a step of subjecting compound 1-b to a reaction with NOTA-bis(t-Bu ester) under the catalytic action of a polypeptide condensation reagent (e.g., an onium salt-type condensation agent; specifically, optionally, HATU, etc.) to give compound 2-a,

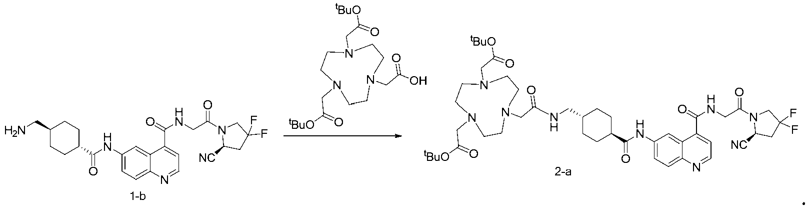

In an alternative embodiment, the aforementioned methods for preparing compounds 5, 7 and 9 or compound 2 each comprise a step of removing a tert-butoxycarbonyl protecting group from compound 1-c under acidic (e.g., hydrochloric acid) or basic conditions to give compound 1-b,

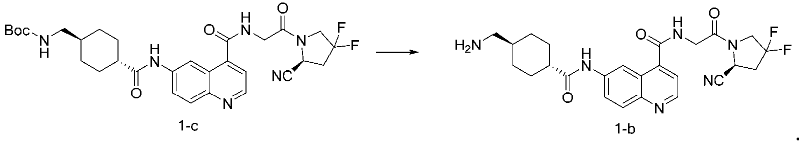

In an alternative embodiment, the aforementioned methods for preparing compounds 5, 7 and 9 or compound 2 each comprise a step of subjecting compounds 1-f and 1-d to a reaction under the action of a condensation agent (e.g., propylphosphonic anhydride) to give compound 1-c,

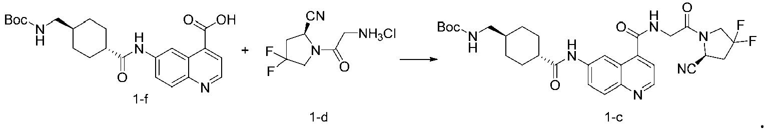

In an alternative embodiment, the aforementioned methods for preparing compounds 5, 7 and 9 or compound 2 each comprise a step of removing a methyl protecting group from compound 1-g under basic conditions (e.g., lithium hydroxide) to give compound 1-f:

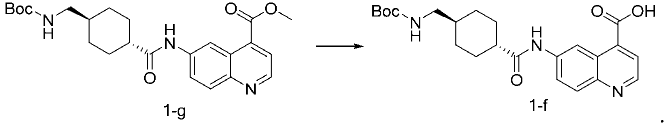

Provided is a method for preparing compound 8 as shown below, 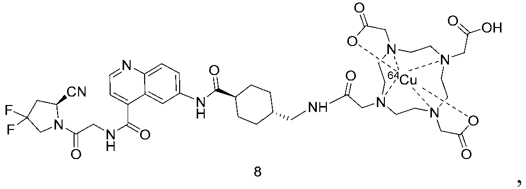 comprising a step of subjecting compound 1 to a reaction with ⁶⁴CuCl₂ under acidic conditions (e.g., acetic acid/sodium acetate buffer) at high temperature (e.g., 75°C-100°C),

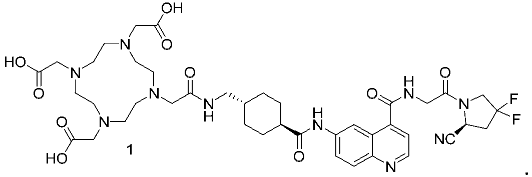

Provided is a method for preparing compound 1, comprising a step of removing a tert-butyl protecting group from compound 1-a under acidic conditions (e.g., trifluoroacetic acid),

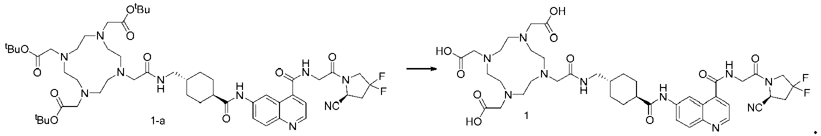

In an alternative embodiment, the aforementioned method for preparing compound 8 or compound 1 comprises a step of subjecting compound 1-b to a reaction with the compound DOTA-tris(t-Bu ester) under the catalytic action of a polypeptide condensation reagent (e.g., an onium salt-type condensation reagent; specifically, optionally, HATU, etc.) to give compound 1-a,

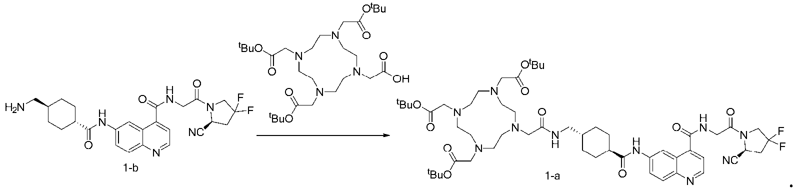

In an alternative embodiment, the aforementioned method for preparing compound 8 or compound 1 comprises a step of removing a tert-butoxycarbonyl protecting group from compound 1-c under acidic (e.g., hydrochloric acid) or basic conditions to give compound 1-b,

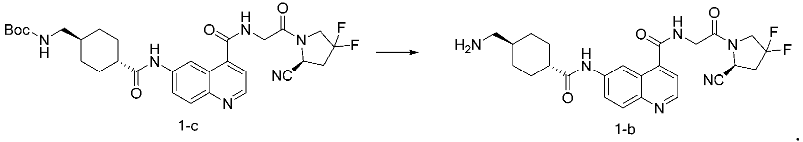

In an alternative embodiment, the aforementioned method for preparing compound 8 or compound 1 comprises a step of subjecting compounds 1-f and 1-d to a reaction under the action of a condensation agent (e.g., propylphosphonic anhydride) to give compound 1-c,

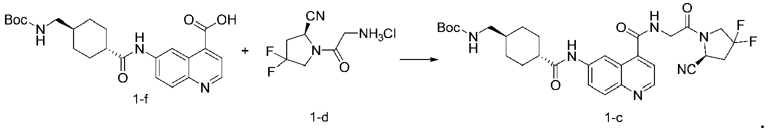

In an alternative embodiment, the aforementioned method for preparing compound 8 or compound 1 comprises a step of removing a methyl protecting group from compound 1-g under basic conditions (e.g., lithium hydroxide) to give compound 1-f:

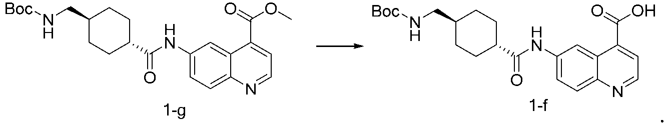

The present disclosure further provides compounds as shown below:

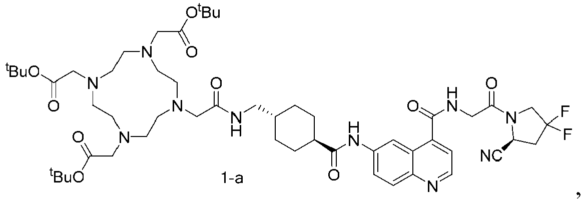

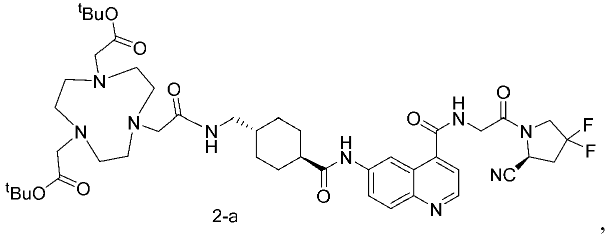

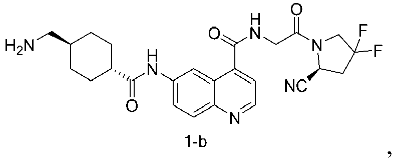

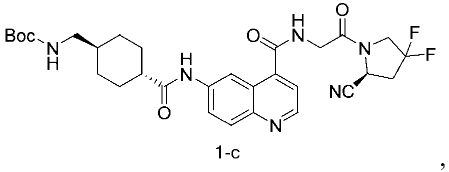

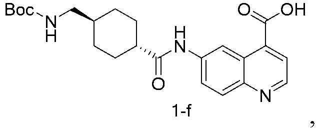

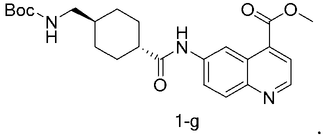

In the compound represented by formula (I) in the present disclosure 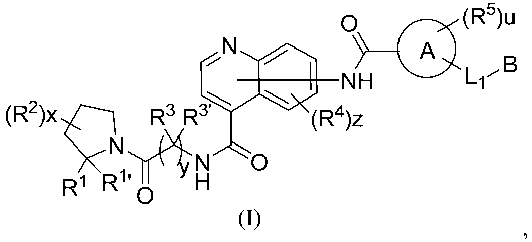 the representation of the long bond indicates that 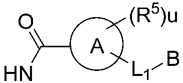 can be attached to any position on the quinoline ring except the 1- and 4-positions.

In the present disclosure, the representation of the attachment between the chelating agent and the radioactive element in the structure of the radiolabelled compound is not limited to one type. Taking compound 7 as an example, 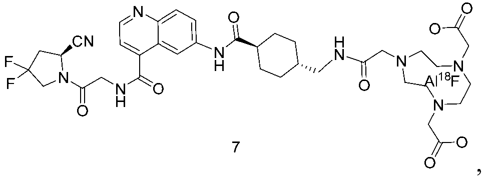 the structural formula may also be represented as: 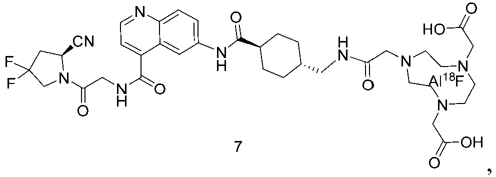 or 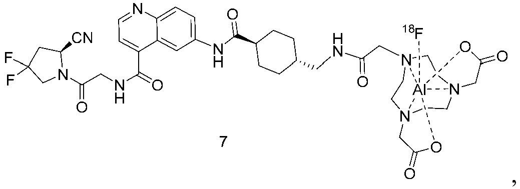 wherein 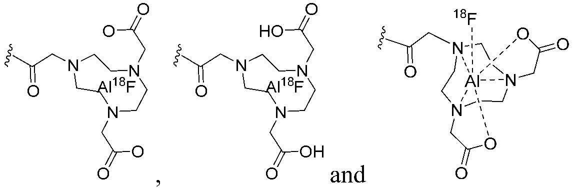 all indicate that a coordinate bond is formed between the chelating agent and A1¹⁸F.

The pharmaceutically acceptable salts of the compounds described in the present disclosure may be selected from inorganic salts or organic salts.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in substituents such as alkyl. All these isomers and mixtures thereof are encompassed within the scope of the present disclosure. The compound comprising asymmetric carbon atoms of the present disclosure can be isolated in optically active-pure or racemic forms. The optically active-pure form can be resolved from the racemic mixture or synthesized by using chiral raw materials or chiral reagents.

Optically active (*R*)- and (*S*)-isomers and *D* and *L* isomers can be prepared by using chiral synthesis or chiral reagents or other conventional techniques. If an enantiomer of a compound of the present disclosure is desired, it can be prepared by asymmetric synthesis or derivatization with a chiral auxiliary, wherein the resulting diastereomeric mixture is separated and the auxiliary groups are cleaved to provide pure desired enantiomers. Alternatively, where the molecule contains a basic functional group (such as amino) or an acidic functional group (such as carboxy), diastereomeric salts can be formed with an appropriate optically active acid or base, followed by resolution of the diastereomers using conventional methods well known in the art, and subsequent recovery of the pure enantiomers. In addition, separation of enantiomers and diastereomers is generally accomplished by using chromatography, which uses chiral stationary phases, optionally in combination with chemical derivatization methods (e.g., formation of carbamates from amines).

In the chemical structures of the compounds of the present disclosure, a bond " 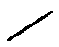 " represents an unspecified configuration; that is, if chiral isomers exist in the chemical structures, the bond " 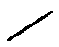 " may be " 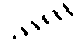 " or " 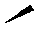 ", or both the configurations of " 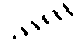 " and " 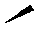 " are included simultaneously. In the chemical structures of the compounds of the present disclosure, a bond " 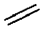 " does not specify a configuration; that is, the configuration for the bond " 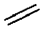 " can be an E configuration or a Z configuration, or both the configurations of E and Z are included simultaneously.

The compounds and intermediates of the present disclosure may also exist in different tautomeric forms, and all such forms are encompassed within the scope of the present disclosure. The term "tautomer" or "tautomeric form" refers to structural isomers of different energies which are interconvertible via a low energy barrier. For example, proton tautomers (also known as proton transfer tautomers) include interconversion via proton migration, such as keto-enol, imine-enamine and lactam-lactim isomerization. An example of a lactam-lactim equilibrium is present between A and B as shown below.

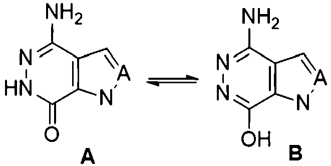

All compounds in the present disclosure may be drawn as form A or form B. All tautomeric forms are encompassed within the scope of the present disclosure. The names of the compounds do not exclude any tautomers.

The present disclosure also includes isotopically-labelled compounds of the present disclosure that are identical to those recited herein, but have one or more atoms replaced by an atom having an atomic mass or mass number different from the atomic mass or mass number usually found in nature. Examples of isotopes that can be incorporated into compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively.

Unless otherwise stated, when a position is specifically designated as deuterium (D), the position should be construed as having deuterium at an abundance at least 1000 times greater than the natural abundance of deuterium (which is 0.015%) (i.e., at least 10% incorporation of deuterium). The expression "having deuterium at an abundance greater than the natural abundance of deuterium" in the compounds in examples means having deuterium at an abundance at least 1000 times greater, at least 2000 times greater, at least 3000 times greater, at least 4000 times greater, at least 5000 times greater, at least 6000 times greater, or having deuterium at a higher abundance. The present disclosure further includes various deuterated forms of the compound of formula (I). Each available hydrogen atom attached to a carbon atom can be independently replaced by a deuterium atom. Those skilled in the art can synthesize deuterated forms of the compound of formula (I) by referring to relevant literatures. The deuterated forms of the compound of formula (I) can be prepared using commercially available deuterated starting materials, or can be synthesized using deuterated reagents using conventional techniques. The deuterated reagents include, but are not limited to, deuterated borane, solution of trideuterated borane in tetrahydrofuran, deuterated lithium aluminium hydride, deuterated iodoethane and deuterated iodomethane.

"Optionally" or "optional" means that the event or circumstance subsequently described may but need not occur, and the description includes the case where the event or circumstance occurs or does not occur. For example, "C₁₋₆ alkyl optionally substituted with halogen or cyano" means that halogen or cyano may but need not be present. The description includes the case where the alkyl is substituted with halogen or cyano and the case where the alkyl is not substituted with halogen or cyano.

### Explanation of terms:

"Pharmaceutical composition" denotes a mixture containing one or more compounds described herein or physiologically and pharmaceutically acceptable salts or prodrugs thereof and other chemical components, as well as other components, such as physiologically and pharmaceutically acceptable carriers and excipients. The purpose of pharmaceutical compositions is to facilitate administration to living organisms and facilitate the absorption of active ingredients to exert biological activity.

"Pharmaceutically acceptable excipient" includes, but is not limited to, any adjuvant, carrier, glidant, sweetener, diluent, preservative, dye/colorant, flavouring agent, surfactant, wetting agent, dispersant, suspending agent, stabilizer, isotonic agent, solvent, or emulsifier that has been approved by the U.S. Food and Drug Administration (FDA) as acceptable for use in humans or livestock animals.

"Effective amount" or "therapeutically effective amount" described in the present disclosure includes an amount sufficient to ameliorate or prevent a symptom or disorder of a medical disorder. An effective amount also means an amount sufficient to allow or facilitate diagnosis. The effective amount for a particular patient or veterinary subject may vary depending on the following factors: for example, the condition to be treated, the patient's general health, the method, route and dosage of administration, and the severity of side effects. An effective amount may be the maximum dosage or dosage regimen that avoids significant side effects or toxic effects.

"Alkyl" refers to a linear or branched saturated aliphatic hydrocarbon group comprising 1 to 20 carbon atoms, for example, alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl and various branched-chain isomers thereof, etc.

"Alkenyl" refers to an unsaturated aliphatic linear or branched hydrocarbon group containing one or more carbon-carbon double bonds. Exemplary alkenyl groups include C₂-C₈, C₂-C₇, C₂-C₆, C₂-C₄, C₃-C₁₂ and C₃-C₆ alkenyl groups, including but not limited to, vinyl (i.e., vinyl), 1-propenyl, 2-propenyl (i.e., allyl), 2-methyl-1-propenyl, 1-butenyl, 2-butenyl (i.e., crotyl), etc.

"Alkynyl" refers to an unsaturated aliphatic linear or branched hydrocarbon group containing one or more carbon-carbon triple bonds. Exemplary alkenyl groups include C₂-C₈, C₂-C₇, C₂-C₆, C₂-C₄, C₃-C₁₂ and C₃-C₆ alkynyl groups, including but not limited to, ethynyl, prop-1-ynyl, but-1-ynyl, pent-1-ynyl, pent-4-ynyl and pent-1,4-diynyl. Alkenyl groups used in any context herein is optionally substituted in the same manner as alkyl groups.

The term "cycloalkyl" refers to a saturated or partially unsaturated, monocyclic or polycyclic hydrocarbon substituent. The cycloalkyl ring comprises 3 to 12 carbon atoms, preferably 4 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Examples of polycyclic cycloalkyl include spiro ring, fused ring and bridged ring cycloalkyl.

The term "heterocycloalkyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen or S(O)ₘ (wherein m is an integer of 0 to 2), but excluding ring moieties of -O-O-, -O-S- or -S-S-, and the remaining ring atoms are carbon. Preferably, the heterocycloalkyl comprises 3 to 12 ring atoms, of which 1-4 are heteroatoms; more preferably, the heterocycloalkyl comprises 3 to 7 ring atoms. Non-limiting examples of "heterocycloalkyl" include: 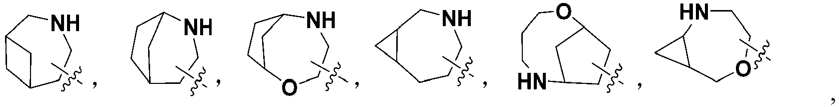 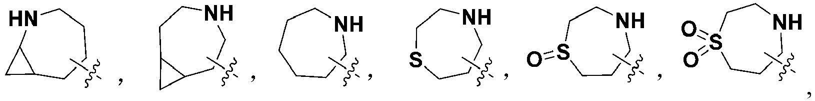 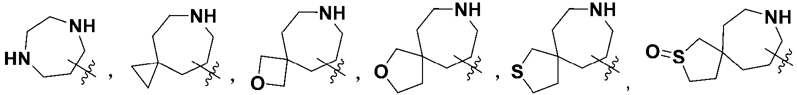 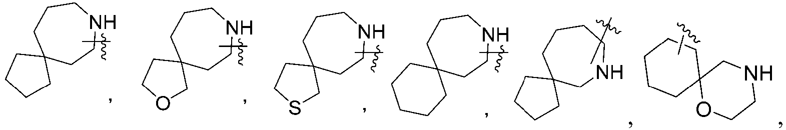 etc.

The heterocycloalkyl ring may be fused to an aryl or heteroaryl ring, wherein the ring attached to the parent structure is heterocycloalkyl, and the non-limiting examples thereof include: 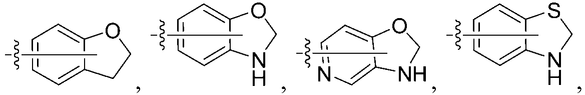 etc.

The heterocycloalkyl may be optionally substituted or unsubstituted.

The term "alkoxy" refers to -O-(alkyl), wherein the alkyl is as defined above. Non-limiting examples of alkoxy include: methoxy, ethoxy, propoxy and butoxy.

The term "alkylthio" refers to -S-(alkyl), wherein the alkyl is as defined above. Non-limiting examples of alkoxy include: methylthio, ethylthio, propylthio and butylthio.

"Monovalent group" refers to a group formed by "formally" eliminating a monovalent atom or group from a compound. "-ylene" refers to a group formed by "formally" eliminating two monovalent atoms or atomic groups or one divalent atom or atomic group from a compound.

The term "alkylene" refers to the moiety remaining after removal of two hydrogen atoms from an alkane molecule, including linear and branched -ylene groups comprising 1 to 20 carbon atoms. Non-limiting examples of alkylene containing 1 to 6 carbon atoms include methylene (-CH₂-) and ethylene (such as - CH₂CH₂- or -CH(CH₃)-).

Similarly, the definitions of "alkyleneoxy", "alkenylene", "alkenyleneoxy", "cycloalkylene" and "heterocycloalkylene" are as defined for "alkylene".

The term "aryl" refers to a 6- to 14-membered all-carbon monocyclic or fused polycyclic (i.e., rings sharing an adjacent pair of carbon atoms) group having a conjugated π electron system, preferably 6- to 12-membered aryl, e.g., phenyl and naphthyl. The aryl ring may be fused to a heteroaryl, heterocycloalkyl or cycloalkyl ring, wherein the ring attached to the parent structure is an aryl ring, and the non-limiting examples thereof include:

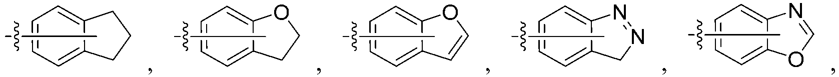

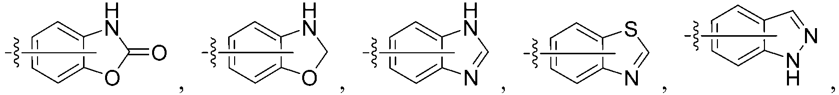

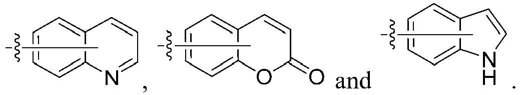

The aryl may be substituted or unsubstituted, and when it is substituted, the substituent is preferably one or more groups independently selected from halogen, hydroxyl, oxo, nitro, cyano, C₁₋₆ alkyl, C₁₋₆ alkoxy, C₂₋₆ alkenyloxy, C₂₋₆ alkynyloxy, 3- to 6-membered cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, 3- to 6-membered cycloalkoxy, 3- to 6-membered heterocycloalkoxy, 3- to 8-membered cycloalkenyloxy, and 5- to 6-membered aryl or heteroaryl are optionally substituted with one or more selected from halogen, hydroxyl, cyano, amino, C₁₋₆ alkyl or C₁₋₆ alkoxy.

The term "heteroaryl" refers to a heteroaromatic system containing 1 to 4 heteroatoms and 5 to 14 ring atoms, wherein the heteroatoms are selected from oxygen, sulfur and nitrogen. The heteroaryl is preferably 6- to 12-membered, and more preferably 5- to 6-membered. For example. Non-limiting examples thereof include: imidazolyl, furyl, thienyl, thiazolyl, pyrazolyl, oxazolyl, isoxazolyl, pyrrolyl, tetrazolyl, pyridyl, pyrimidinyl, thiadiazolyl, pyrazinyl, triazolyl, indazolyl, benzimidazolyl, 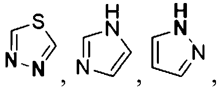 etc.

The heteroaryl ring may be fused to an aryl, heterocycloalkyl or cycloalkyl ring, wherein the ring attached to the parent structure is a heteroaryl ring, and the non-limiting examples thereof include:

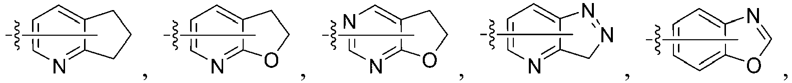

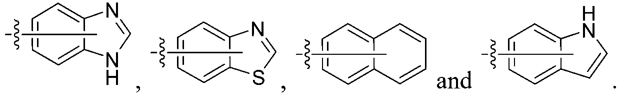

The heteroaryl may be optionally substituted or unsubstituted. When the heteroaryl is substituted, the substituent is preferably one or more groups independently selected from halogen, hydroxyl, cyano, amino, C₁₋₆ alkyl or C₁₋₆ alkoxy.

The term "spiro ring" refers to a compound in which two rings share one atom. Non-limiting examples of spirocycloalkyl include:

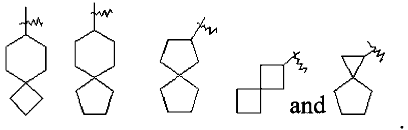

The term "fused ring" refers to a compound formed by fusing two or more rings by sharing two adjacent atoms. Non-limiting examples of fused cycloalkyl include:

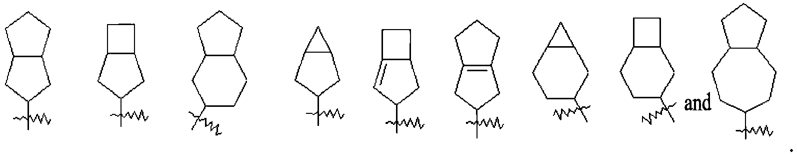

The term "bridged ring" refers to a structure formed by two or more cyclic structures sharing two non-adjacent ring atoms with each other. According to the number of constituent rings, it can be divided into bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

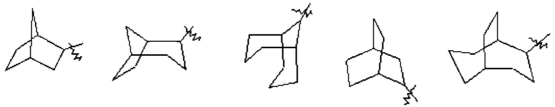

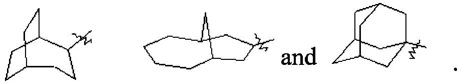

The term "heterocycle" refers to a ring containing, in addition to carbon atoms, other kinds of atoms, and includes heterocycloalkyl and heteroaromatic rings.

The term "hydroxyl" refers to -OH group.

The term "halogen" refers to fluorine, chlorine, bromine or iodine.

The term "cyano" refers to -CN.

The term "amino" refers to -NH₂.

The term "nitro" refers to -NO₂.

The term "oxo" refers to the =O substituent.

"Substituted" refers to one or more hydrogen atoms in the group, preferably at most 5, more preferably 1-3 hydrogen atoms each independently substituted with a corresponding number of substituents. When the substituent is a ketone or oxo (i.e., =O), two (2) hydrogens on the atom are replaced.
DOTA-tris(t-Bu ester): 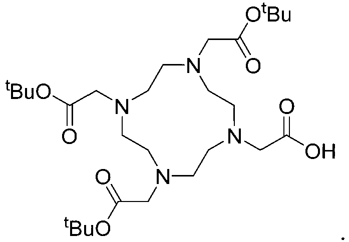
NOTA-bis(t-Bu ester): 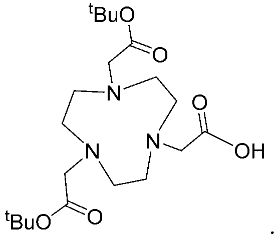
HATU: 2-(7-azabenzotriazol-1-yl)-N,N,N',N'-tetramethyluronium hexafluorophosphate.
T3P: propylphosphonic anhydride.

### Detailed Description of Embodiments

The present disclosure will be further described below in conjunction with examples, but these examples are not intended to limit the scope of the present disclosure.

Experimental methods without specific conditions indicated in the examples of the present disclosure are generally conducted under conventional conditions, or conditions recommended by the manufacturers of the starting materials or commercial products. The reagents for which the specific source is not indicated, are conventional commercially available reagents.

The chromatographic conditions for HPLC/MS analysis in the present disclosure are as follows:
10 µl of each sample is injected automatically. Mobile phase: A: 0.1% formic acid in water, B: 0.1% formic acid in acetonitrile. Flow rate: 1.5 ml/min. Gradient: from 10% B to 95% B in 0-6 min, from 95% B to 100% B in 6-8 min, from 100% B to 10% B in 8-8.10 min, and 10% B maintained in 8.10-11.0 min.
Equipment model: Thermo Scientific ULTIMATE3000 ISQEM.
Chromatographic column: Eclipse Plus C18, 3.5 nm, 4.6 × 100 mm.
UV detection wavelength: 254 nM.

Compound purity data is obtained by manual integration, and the molecular weight [M+1]⁺ is collected.

The chromatographic conditions for preparative liquid chromatography in the present disclosure are as follows:
Mobile phase: A: 0.1% trifluoroacetic acid in water, B: 0.1% trifluoroacetic acid in acetonitrile. Flow rate: 16 ml/min. Gradient: from 25% B to 35% B in 0-25.0 min, from 35% B to 70% B in 25.0-25.1 min, and 70% B maintained in 25.1-33.0 min.
Equipment model: Agilent AGILENT1260II.
Chromatographic column: HPLCONE, 5.0 µm, 30 × 250 mm.
UV detection wavelength: 254 nM. The target compound is collected and lyophilized.

### Example 1. Preparation of 6-(trans-4-(((2-(4,7,10-tricarboxymethyl-1,4,7,10-tetraazacyclododecan-1-yl)acetyl)amino)methyl)cyclohexan-1-yl)carboxamido-4-((S)-2-(2-cyano-4,4-difluoropyrrolidin-1-yl)-2-oxoethyl)carbamoylquinoline (1)

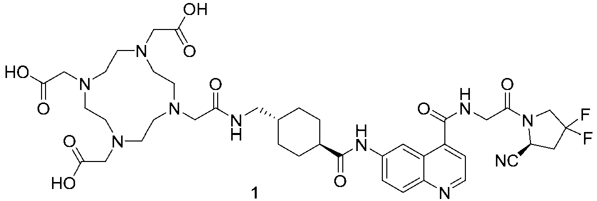

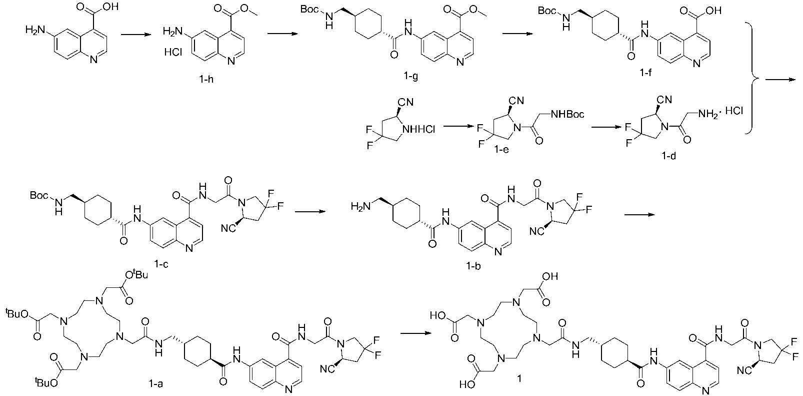

### Step 1. Preparation of methyl 6-aminoquinoline-4-carboxylate hydrochloride (1-h)

Under ice bath conditions, 12.5 g of acetyl chloride was slowly added dropwise to 100 ml of methanol. The mixture was stirred at room temperature for 0.5 hours, and then 5.0 g of 6-aminoquinoline-4-carboxylic acid was added. The mixture was heated to reflux for 12 hours. The solvent was evaporated to dryness, and the residue was slurried with isopropyl ether and filtered to give compound 1-h (6.23 g, yield: 98.2%).
MS m/z (ESI): 203.03 [M+1]⁺.

### Step 2. Preparation of methyl 6-(trans-4-(((tert-butoxycarbonyl)amino) methyl)cyclohexan-1-yl)carboxamidoquinoline-4-carboxylate (I-g)

Under ice bath conditions, 500 mg of compound **1-h,** 636 mg of trans-4-(tert-butoxycarbonylaminomethyl)cyclohexanecarboxylic acid, 2832 mg of a solution of propylphosphonic anhydride in ethyl acetate (T3P, 50% mass concentration) and 959 mg of N,N-diisopropylethylamine were added to 30 ml of tetrahydrofuran. The mixture was reacted at 35°C for 8 hours. The reaction solution was poured into 0.5 N hydrochloric acid and extracted with ethyl acetate. The organic phase was washed with a saturated sodium bicarbonate solution and dried over anhydrous sodium sulfate, and then the solvent was evaporated to dryness to give compound **1-g** (887 mg, yield: 95.9%).
MS m/z (ESI): 442.32 [M+1]⁺.

### Step 3. Preparation of 6-(trans-4-(((tert-butoxycarbonyl)amino) methyl)cyclohexan-1-yl)carboxamidoquinoline-4-carboxylic acid (1-f)

600 mg of compound **1-g** was added to 20 ml of a mixed solvent of tetrahydrofuran/water (1 : 1), and then 171 mg of lithium hydroxide monohydrate was added. The mixture was reacted at room temperature for 3 hours. Tetrahydrofuran in the solvent was evaporated to dryness, and the pH was adjusted to 3-4 with 0.5 N hydrochloric acid, with a large amount of solid precipitated. The mixture was filtered to give compound **1-f** (543 mg, yield: 93.6%).
MS m/z (ESI): 428.11 [M+1]⁺

### Step 4. Preparation of (S)-1-(2-((tert-butoxycarbonyl)amino)acetyl)-4,4-difluoropyrrolidine-2-carbonitrile (1-e)

Under ice bath conditions, 5.0 g of (S)-4,4-difluoropyrrolidine-2-carbonitrile hydrochloride, 5.0 g of Boc-glycine, 31.5 g of a solution of propylphosphonic anhydride in ethyl acetate (T3P, 50% mass concentration) and 10.68 g of N,N-diisopropylethylamine were added to 100 ml of tetrahydrofuran. The mixture was reacted at 35°C for 5 hours. The reaction solution was poured into 0.5 N hydrochloric acid and extracted with ethyl acetate. The organic phase was washed with a saturated sodium bicarbonate solution and dried over anhydrous sodium sulfate, and then the solvent was evaporated to dryness to give compound **1-e** (7.8 g, yield: 90.4%).
MS m/z (ESI): 290.08 [M+1]⁺.

### Step 5. Preparation of (S)-1-aminoacetyl-4,4-difluoropyrrolidine-2-carbonitrile (1-d)

7.8 g of compound **1-e** was dissolved in 100 ml of a 3.0 mol/L solution of hydrochloric acid in ethyl acetate. The mixture was stirred at room temperature for 5 hours, and then the solvent was evaporated to dryness to give compound **1-d** (6.07 g, yield: 98.7%).
MS m/z (ESI): 190.13 [M+1]⁺.

### Step 6. Preparation of 6-(trans-4-(((tert-butoxycarbonyl)amino)methyl)cyclohexan-1-yl)carboxamido-4-((S)-2-(2-cyano-4,4-difluoropyrrolidin-1-yl)-2-oxoethyl)carbamoylquinoline (I-c)

Under ice bath conditions, 500 mg of compound **1-f,** 221 mg of **1-d,** 1340 mg of a solution of propylphosphonic anhydride (T3P) in ethyl acetate (T3P, 50% mass concentration) and 453 mg of N,N-diisopropylethylamine were added to 20 ml of tetrahydrofuran. The mixture was reacted at 35°C for 5 hours. The reaction solution was poured into 0.5 N hydrochloric acid and extracted with ethyl acetate. The organic phase was washed with a saturated sodium bicarbonate solution and dried over anhydrous sodium sulfate, and then the solvent was evaporated to dryness to give compound **1-c** (572 mg, yield: 81.7%).
MS m/z (ESI): 599.10 [M+1]⁺.

### Step 7. Preparation of 6-(trans-4-(aminomethyl)cyclohexan-1-yl)carboxamido-4-((S)-2-(2-cyano-4,4-difluoropyrrolidin-1-yl)-2-oxoethyl)carbamoylquinoline (1-b)

572 mg of compound **1-c** was dissolved in 30 ml of a 3.0 mol/L solution of hydrochloric acid in ethyl acetate. The mixture was stirred at room temperature for 5 hours, and then the solvent was evaporated to dryness to give compound **1-b** (461 mg, yield: 96.7%).
MS m/z (ESI): 499.13 [M+1]⁺.

### Step 8. Preparation of 6-(trans-4-(((2-(4,7,10-tri-tert-butoxycarbonylmethyl-1,4,7,10-tetraazacyclododecan-1-yl)acetyl)amino) methyl)cyclohexan-1-yl)carboxamido-4-((S)-2-(2-cyano-4,4-difluoropyrrolidine-1-yl)-2-oxoethyl)carbamoylquinoline (1-a)

300 mg of compound **1-b,** 413 mg of tri-tert-butyl 1,4,7,10-tetraazacyclododecane-1,4,7,10-tetraacetate (DOTA-tris(t-Bu ester)), 412 mg of HATU and 233 mg of N,N-diisopropylethylamine were dissolved in tetrahydrofuran. The mixture was reacted at 35°C for 5 hours. The reaction solution was poured into water and extracted with dichloromethane. The organic phase was dried over anhydrous sodium sulfate. The filtrate was concentrated under reduced pressure and purified by silica gel column chromatography eluting with an eluent system of dichloromethane/methanol (methanol: 0% to 20%) to give compound **1-a** (464 mg, yield: 73.2%).
MS m/z (ESI): 1053.46 [M+1]⁺.

### Step 9. Preparation of 6-(trans-4-(((2-(4,7,10-tricarboxymethyl-1,4,7,10-tetraazacyclododecan-1-yl)acetyl)amino)methyl)cyclohexan-1-yl)carboxamido-4-((S)-2-(2-cyano-4,4-difluoropyrrolidin-1-yl)-2-oxoethyl)carbamoylquinoline (1)

464 mg of compound **1-a** was dissolved in 20 ml of dichloromethane, and then 20 ml of trifluoroacetic acid was added. The mixture was stirred at room temperature overnight, and then the solvent was evaporated to dryness. The residue was separated and purified by preparative liquid phase chromatography, and then lyophilized to give the target compound **1.** (166 mg, yield: 42.5%).
MS m/z (ESI): 885.36 [M+1]⁺.

### Example 2. Preparation of 6-(trans-4-(((2-(4,7-dicarboxymethyl-1,4,7-triazacyclononan-1-yl)acetyl)amino)methyl)cyclohexan-1-yl)carboxamido-4-((S)-2-(2-cyano-4,4-difluoropyrrolidin-1-yl)-2-oxoethyl)carbamoylquinoline (2)

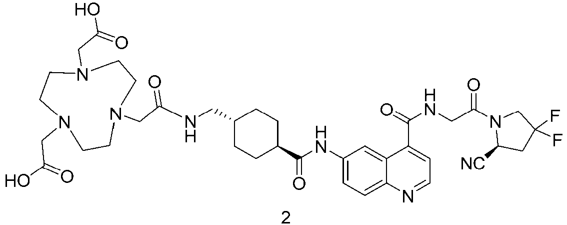

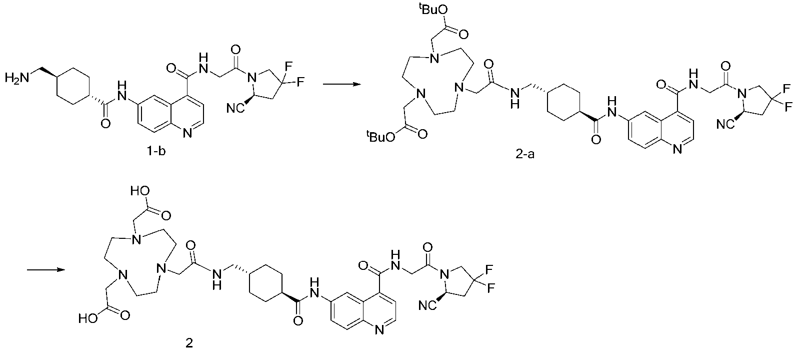

Compound **2** was prepared with reference to the same method as that for compound **1.**

### Step 1. Preparation of 6-(trans-4-(((2-(4,7-di-tert-butoxycarbonylmethyl-1,4,7-triazacyclononan-1-yl)acetyl)amino)methyl)cyclohexan-1-yl)carboxamido-4-((S)-2-(2-cyano-4,4-difluoropyrrolidin-1-yl)-2-oxoethyl)carbamoylquinoline (2-a)

300 mg of compound **1-b,** 300 mg of di-tert-butyl 1,4,7-triazacyclononane-1,4,7-triacetate (NOTA-bis(t-Bu ester)), 412 mg of HATU and 233 mg of N,N-diisopropylethylamine were dissolved in tetrahydrofuran. The mixture was reacted at 35°C for 5 hours. The reaction solution was poured into water and extracted with dichloromethane. The organic phase was dried over anhydrous sodium sulfate. The filtrate was concentrated under reduced pressure and purified by silica gel column chromatography eluting with an eluent system of dichloromethane/methanol (methanol: 0% to 20%) to give compound **2-a** (368 mg, yield: 68.2%).
MS m/z (ESI): 896.33 [M+1]⁺.

### Step 2. Preparation of 6-(trans-4-(((2-(4,7-dicarboxymethyl-1,4,7-triazacyclononan-1-yl)acetyl)amino)methyl)cyclohexan-1-yl)carboxamido-4-((S)-2-(2-cyano-4,4-difluoropyrrolidin-1-yl)-2-oxoethyl)carbamoylquinoline (2)

368 mg of compound **2-a** was dissolved in 20 ml of dichloromethane, and then 20 ml of trifluoroacetic acid was added. The mixture was stirred at room temperature overnight, and then the solvent was evaporated to dryness. The residue was separated and purified by preparative liquid phase chromatography, and then lyophilized to give the target compound **2** (125 mg, yield: 38.7%).

¹H NMR (400 MHz, DMSO-d₆) δ 10.30 (s, 1H), 9.10(t, 1H), 8.90 (d, 1H), 8.57 (s, 1H), 8.25 (t, 1H), 8.03 (m, 2H), 7.59 (d, 1H), 5.15 (dd, 1H), 4.11-4.34 (m, 4H), 3.78 (d, 2H), 3.62(s, 4H), 2.81-3.01(m, 16H), 2.34-2.40 (m, 1H), 1.80-1.92 (m, 4H), 1.40-1.48 (m, 3H), 0.93-1.02 (m, 2H).
MS m/z (ESI): 784.10 [M+1]⁺.

### Example 3. Preparation of 6-((6r,9r)-N-(2-(4,7,10-tricarboxymethyl-1,4,7,10-tetraazacyclododecan-1-yl)acetyl)-1-oxa-4-azaspiro[5.5]undecan-9-yl)carboxamido-4-((S)-2-(2-cyano-4,4-difluoropyrrolidin-1-yl)-2-oxoethyl)carbamoylquinoline (3)

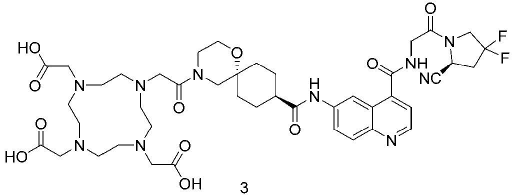

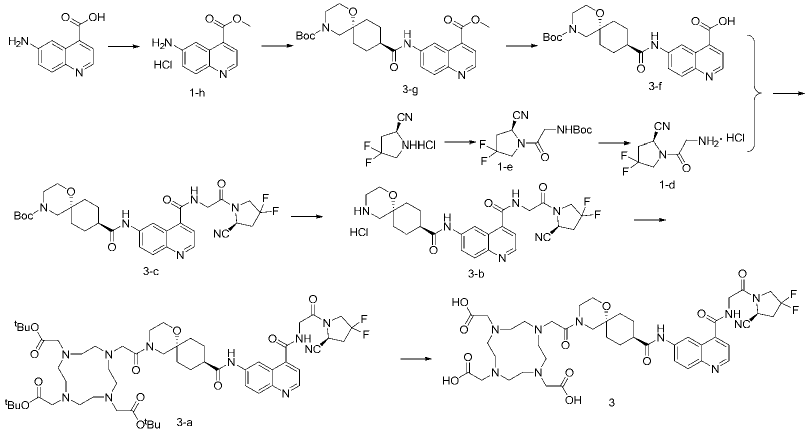

Compounds **1-h** and **1-d** were prepared with reference to the method for compound **1.**

### Step 1. Preparation of methyl 6-((6r,9r)-N-tert-butoxycarbonyl-1-oxa-4-azaspiro[5.5]undecan-9-yl)carboxamidoquinoline-4-carboxylate (3-g)

Under ice bath conditions, 500 mg of compound **1-h,** 528 mg of (6r,9r)-N-Boc-1-oxa-4-azaspiro[5.5]undecane-9-carboxylic acid, 2832 mg of a solution of propylphosphonic anhydride in ethyl acetate (T3P, 50% mass concentration) and 959 mg of N,N-diisopropylethylamine were added to 30 ml of tetrahydrofuran. The mixture was reacted at 35°C for 8 hours. The reaction solution was poured into 0.5 N hydrochloric acid and extracted with ethyl acetate. The organic phase was washed with a saturated sodium bicarbonate solution and dried over anhydrous sodium sulfate, and then the solvent was evaporated to dryness to give compound **3-g** (815 mg, yield: 93.2%).
MS m/z (ESI): 484.35 [M+1]⁺.

### Step 2. Preparation of 6-((6r,9r)-N-tert-butoxycarbonyl-1-oxa-4-azaspiro[5.5]undecan-9-yl)carboxamidoquinoline-4-carboxylic acid (3-f)

815 mg of compound **3-g** was added to 20 ml of a mixed solvent of tetrahydrofuran/water (1:1), and then 214 mg of lithium hydroxide monohydrate was added. The mixture was reacted at room temperature for 3 hours. Tetrahydrofuran in the solvent was evaporated to dryness, and the pH was adjusted to 3-4 with 0.5 N hydrochloric acid, with a large amount of solid precipitated. The mixture was filtered to give compound **3-f** (543 mg, yield: 95.7%) MS m/z (ESI): 470.31[M+1]⁺.

### Step 3. Preparation of 6-((6r,9r)-N-tert-butoxycarbonyl-1-oxa-4-azaspiro[5.5]undecan-9-yl)carboxamido-4-((S)-2-(2-cyano-4,4-difluoropyrrolidin-1-yl)-2-oxoethyl)carbamoylquinoline (3-c)

Under ice bath conditions, 500 mg of compound **3-f,** 240 mg of **1-d,** 1340 mg of a solution of propylphosphonic anhydride in ethyl acetate (T3P, 50% mass concentration) and 453 mg of N,N-diisopropylethylamine were added to 20 ml of tetrahydrofuran. The mixture was reacted at 35°C for 5 hours. The reaction solution was poured into 0.5 N hydrochloric acid and extracted with ethyl acetate. The organic phase was washed with a saturated sodium bicarbonate solution and dried over anhydrous sodium sulfate, and then the solvent was evaporated to dryness to give compound **3-c** (521 mg, yield: 76.3%) MS m/z (ESI): 641.29 [M+1]⁺.

### Step 4. Preparation of 6-((6r,9r)-1-oxa-4-azaspiro[5.5]undecan-9-yl)carboxamido-4-((S)-2-(2-cyano-4,4-difluoropyrrolidin-1-yl)-2-oxoethyl)carbamoylquinoline (3-b)

521 mg of compound **3-c** was dissolved in 30 ml of a 3.0 mol/L solution of hydrochloric acid in ethyl acetate. The mixture was stirred at room temperature for 5 hours, and then the solvent was evaporated to dryness to give compound **3-b** (619 mg, yield: 94.8%).
MS m/z (ESI): 541.24 [M+1]⁺.

### Step 5. Preparation of 6-((6r,9r)-N-(2-(4,7,10-tri-tert-butoxycarbonylmethyl-1,4,7,10-tetraazacyclododecan-1-yl)acetyl)-1-oxa-4-azaspiro[5.5]undecan-9-yl)carboxamido-4-((S)-2-(2-cyano-4,4-difluoropyrrolidin-1-yl)-2-oxoethyl)carbamoylquinoline (3-a)

300 mg of compound **3-b,** 357 mg of tri-tert-butyl 1,4,7,10-tetraazacyclododecane-1,4,7,10-tetraacetate (DOTA-tris(t-Bu ester)), 356 mg of HATU and 202 mg of N,N-diisopropylethylamine were dissolved in 20 ml of tetrahydrofuran. The mixture was reacted at 35°C for 5 hours. The reaction solution was poured into water and extracted with dichloromethane. The organic phase was dried over anhydrous sodium sulfate. The filtrate was concentrated under reduced pressure and purified by silica gel column chromatography eluting with an eluent system of dichloromethane/methanol (methanol: 0% to 20%) to give compound **3-a** (395 mg, yield: 69.4%).
MS m/z (ESI): 1095.58 [M+1]⁺.

### Step 6. Preparation of 6-((6r,9r)-N-(2-(4,7,10-tricarboxymethyl-1,4,7,10-tetraazacyclododecan-1-yl)acetyl)-1-oxa-4-azaspiro[5.5]undecan-9-yl)carboxamido-4-((S)-2-(2-cyano-4,4-difluoropyrrolidin-1-yl)-2-oxoethyl)carbamoylquinoline (3)

395 mg of compound **3-a** was dissolved in 20 ml of dichloromethane, and then 20 ml of trifluoroacetic acid was added. The mixture was stirred at room temperature overnight, and then the solvent was evaporated to dryness. The residue was separated and purified by preparative liquid phase chromatography, and then lyophilized to give the target compound 3 (89 mg, yield: 26.6%).
MS m/z (ESI): 927.34 [M+1]⁺.

### Example 4. Preparation of 6-((6r,9r)-N-(2-(4,7-dicarboxymethyl-1,4,7-triazacyclononan-1-yl)acetyl)-1-oxa-4-azaspiro[5.5]undecan-9-yl)carboxamido-4-((S)-2-(2-cyano-4,4-difluoropyrrolidin-1-yl)-2-oxoethyl)carbamoylquinoline (4)

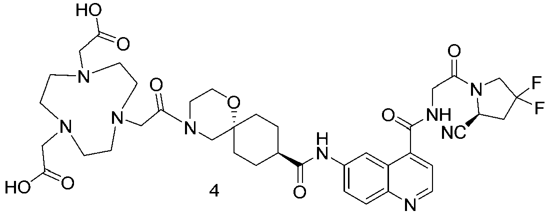

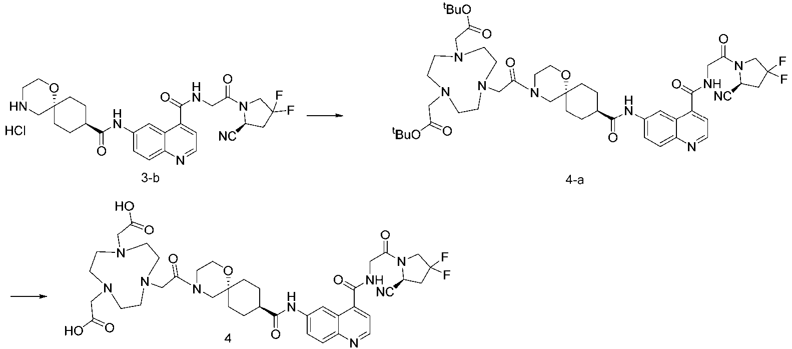

Compound **4** was prepared with reference to the same method as that for compound **3.**

### Step 1. Preparation of 6-((6r,9r)-4-(N-(2-(4,7-di-tert-butoxycarbonylmethyl-1,4,7-triazacyclononan-1-yl)acetyl)-1-oxa-4-azaspiro[5.5]undecan-9-yl)carboxamido-4-((S)-2-(2-cyano-4,4-difluoropyrrolidin-1-yl)-2-oxoethyl)carbamoylquinoline (4-a)

300 mg of compound **3-b,** 259 mg of di-tert-butyl 1,4,7-triazacyclononane-1,4,7-triacetate (NOTA-bis(t-Bu ester)), 356 mg of HATU (1.8 eq) and 202 mg of N,N-diisopropylethylamine were dissolved in 20 ml of tetrahydrofuran. The mixture was reacted at 35°C for 5 hours. The reaction solution was poured into water and extracted with dichloromethane. The organic phase was dried over anhydrous sodium sulfate. The filtrate was concentrated under reduced pressure and purified by silica gel column chromatography eluting with an eluent system of dichloromethane/methanol (methanol: 0% to 20%) to give compound **4-a** (386 mg, yield: 79.1) MS m/z (ESI): 938.42 [M+1]⁺.

### Step 2. Preparation of 6-((6r,9r)-4-(N-(2-(4,7-dicarboxymethyl-1,4,7-triazacyclononan-1-yl)acetyl)-1-oxa-4-azaspiro[5.5]undecan-9-yl)carboxamido-4-((S)-2-(2-cyano-4,4-difluoropyrrolidin-1-yl)-2-oxoethyl)carbamoylquinoline (4)

386 mg of compound **4-a** was dissolved in 20 ml of dichloromethane, and then 20 ml of trifluoroacetic acid was added. The mixture was stirred at room temperature overnight, and then the solvent was evaporated to dryness. The residue was separated and purified by preparative liquid phase chromatography, and then lyophilized to give the target compound **4** (179 mg, yield: 52.7%).

¹H NMR (400 MHz, DMSO-d₆) δ 10.29 (d, 1H), 9.07(s, 1H), 8.87 (d, 1H), 8.52 (d, 1H), 8.03 (d, 2H), 7.56 (d, 1H), 5.15 (t, 1H), 4.19-4.45 (m, 7H), 3.58-4.19 (m, 24H), 1.94(m, 2H), 1.67(m, 4H), 1.27 (m, 2H).
MS m/z (ESI): 826.33 [M+1]⁺.

### Example 5. Preparation of compound 5

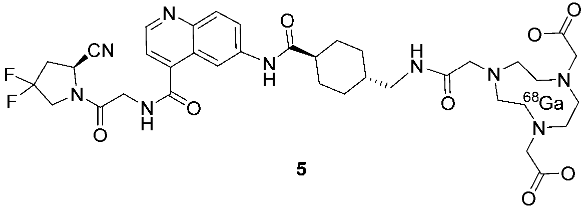

To a reaction flask were added 0.1 ml of sodium acetate buffer, and then 0.9 mL of ⁶⁸GaCl₃ solution (0.1 N hydrochloric acid solution) and 5 µL of a 6 nmol aqueous solution of compound **2.** The reaction solution was maintained at pH 3.0 to 5.0 and heated at 95°C for 10 min-15 min.

The labelled reaction solution was diluted with 2 mL of normal saline. The impurity content was less than 10% as detected by ITLC. The reaction solution was directly used in animal imaging experiments.

### Example 6. Preparation of compound 6

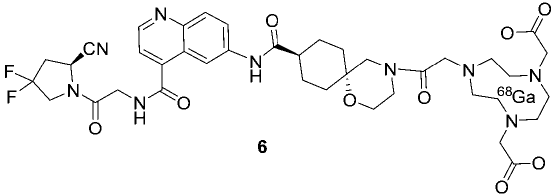

To a reaction flask were added 0.1 ml of sodium acetate buffer, and then 0.9 mL of ⁶⁸GaCl₃ solution (0.1 N hydrochloric acid solution) and 5 µL of a 6 nmol aqueous solution of compound **4.** The reaction solution was maintained at pH 3.0 to 5.0 and heated at 95°C for 10 min-15 min.

The labelled reaction solution was diluted with 2 mL of normal saline. The impurity content was less than 10% as detected by ITLC. The reaction solution was directly used in animal imaging experiments.

### Example 7. Preparation of compound 7

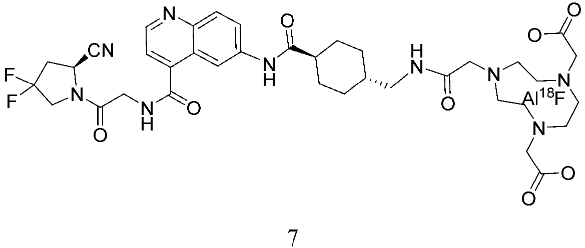

1 µl of a 10 mM aqueous solution of compound 2 was added to 50-100 µl of 0.5 M acetic acid/sodium acetate buffer at pH 4.0-4.4. A 2 mM aqueous solution of AlCl₃ was then added in a molar ratio of 2 : 1, followed by the addition of 50 µl of 1850 MBq QMA-purified ¹⁸F⁻ ions. The mixture was reacted at 100°C for 10-15 min. Purification was performed using an HLB solid-phase extraction cartridge (HLB cartridge). The cartridge was rinsed with ethanol/water (1 : 1), and the eluate was diluted with normal saline until the ethanol content was below 10%. The radiochemical purity was greater than 90% as detected by radio-HPLC. The dilution was directly used in animal imaging experiments.

### Example 8. Preparation of compound 8

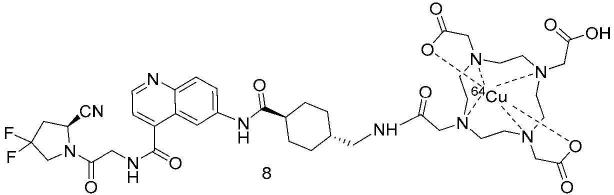

5 µl of a 10 mM aqueous solution of compound 1 was dissolved in 100 µl of acetic acid/sodium acetate buffer solution at pH 3.5-6.5. Then, 50 µl of an 1850 MBq ⁶⁴CuCl₂ solution, which had been dissolved in 0.1 M hydrochloric acid, was added. The mixture was reacted at 80°C for 15 min. Purification was performed by using a C18 column and the eluate was diluted with normal saline until the ethanol content was below 10%. The radiochemical purity was 95.26% as detected by radio-HPLC.

### Example 9. Preparation of compound 9

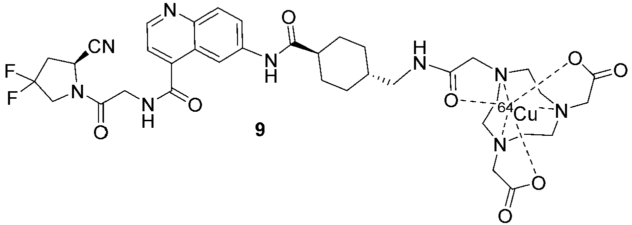

5 µl of a 10 mM aqueous solution of compound 2 was dissolved in 100 µl of acetic acid/sodium acetate buffer solution at pH 3.5-6.5. Then, 50 µl of an 1850 MBq ⁶⁴CuCl₂ solution, which had been dissolved in 0.1 M hydrochloric acid, was added. The mixture was reacted at 80°C for 15 min. Purification was performed by using a C18 column and the eluate was diluted with normal saline until the ethanol content was below 10%. The radiochemical purity was 95.82% as detected by radio-HPLC.

### Biological evaluation

The present disclosure will be further described and explained below in conjunction with test examples, but these examples are not intended to limit the scope of the present disclosure.

### Test Example 1. FAPα enzyme activity test

### 1.1 Experimental materials and instruments

**Table 1. Source information of experimental materials and instruments**

| Experimental materials | Source |
|---|---|
| FAPα protein | TANJIN HENGRUI MEDICINE CO., LTD. |
| Z-Gly-Pro-AMC | GLPBIO, GA23817 |
| PBS | Nanjing SenBeiJia Biological Technology Co., Ltd., BC-BPBS-01 |
| DMSO | sigma, D5879-500ML |
| Microplate | 675076 |
| Microplate reader | TECAN, Spark |

### 1.2 Experimental steps

The substrate (Z-Gly-Pro-AMC) was diluted with DMSO to prepare a 0.5 mM stock solution. Before each experiment, the 0.5 mM stock solution was diluted with PBS to 50 µM for later use. The FAPα protein was diluted with PBS to 0.5 ng/µl for later use. The test compounds and positive control were diluted with PBS to a concentration of 100 nM or 200 nM for the determination of single-point inhibition rate. For compounds with a single-point inhibition rate comparable to that of the positive compound, further determination of IC₅₀ values was performed. The test compounds and positive control were subjected to a 5-fold serial dilution with PBS to obtain 8 gradients for later use, with the highest concentration being 10 µM, and the lowest concentration being 0 nM. Test method: 85 µl of the FAPα dilution and 10 µl of the compound dilution were added to a microplate, uniformly mixed and incubated at 37°C for 10 min. Then, 5 µL of 50 µM substrate was added, uniformly mixed and incubated at 37°C for 10 min. The microplate was read on a microplate reader, with an excitation wavelength of 380 nm and an emission wavelength of 465 nm.

### 1.3 Experimental results

**Table 2.1 Inhibition rate (100 nM) and IC₅₀ of the compounds of the present disclosure against FAPα enzyme**

| Compound No. | Inhibition rate 100 nM | IC₅₀ (nM) |
|---|---|---|
| FAPI-04 | 92.26% | 0.72 |
| Compound 1 | 84.09% | 0.72 |
| Compound 3 | 84.70% | 0.57 |

Using the same method, the IC₅₀ value of compound 2 against FAPα enzyme was determined to be 0.71 times that of the positive drug FAPI-04, indicating comparable efficacy; the IC₅₀ value of FAP-42 against FAPα enzyme was 2.27 times that of the positive drug FAPI-04.

Note: The structure of FAPI-04 is shown as follows:
References: CN 111699181 A, P.60 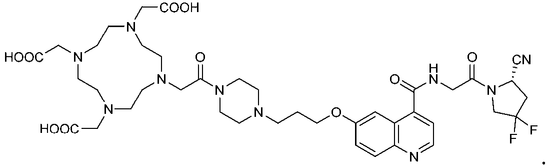
The structure of FAP-42 is shown as follows: CN 111699181 A, P.61

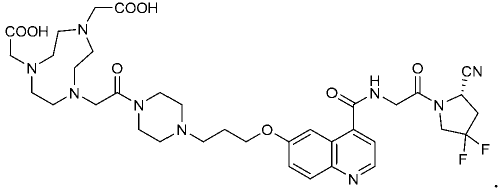

### Test Example 2. PET imaging test of ⁶⁸Ga- and ¹⁸F-labelled compounds

### 2.1 Experimental materials

### Cell:

Cell information: U-87 MG cells (Wuhan Pricella Biotechnology Co., Ltd., Cat. No.: CL-0238, Lot No.: YBMIL8BQHO); Culture conditions: U-87 MG cell specific medium (MEM + 10% FBS + 1% P/S); Number of passages: 6-9;

### Experimental animals:

Germline: BALB/c nude mice; age: 4-5 weeks old; Body weight: 15-22 g;

### Reagents:

PBS (Solarbio, P1020)
Matrigel (ABW, 0827045)
Trypsin-EDTA (Gibco, 25200-072)
U-87 MG specific medium (Pricella, CM-0238)

### Instrument:

Small animal PET/CT (ediso, nanoScan PET/CT 4heads)
Activity meter (Capintec, activity meter)
Electronic balance (Changzhou Shuangjie, DT100)

### 2.2 Experimental steps

### Model construction

A sufficient quantity of U-87 MG cells was prepared and inoculated into the posterior position of the right forelimb of B-NDG mice in an inoculation volume of 100 µL, containing 50% Matrigel and 4 × 10⁶ cells.

### Operation steps

The workbench surface was wiped with 75% medical alcohol and covered with a disposable sterile tablecloth. 0.5 mL insulin syringes, alcohol swabs, cotton swabs and a marking pen were prepared in the injection room. The mice were placed in a mouse restrainer, and the tails of the mice were disinfected with alcohol swabs. 0.2-0.5 mL of the formulated test sample was injected into each mouse via the tail vein, with the time of each injection, the activity of the syringe and the activity of the empty syringe recorded. Tumour-bearing mice were anesthetized with isoflurane, then placed in a prone position on small animal PET beds and fixed. PET static image acquisition was performed for 10 min at 0.5 h, 1 h, 2 h and 4 h after administration; a whole-body CT scan was performed before each static scan to obtain distribution images of the labelled compound throughout the body of tumour-bearing mice. PET images at different time points after administration were obtained for each experimental animal. Major organs were selected and delineated, specifically including: parts such as tumour, muscle, bone, lung, brain, liver and kidney. The radioactive accumulation and clearance of the labelled compounds in tumours and non-target tissues of tumour-bearing mice were observed.

### 3. Experimental results

**Table 3.1 Uptake of the compounds of the present disclosure in tumour (tissues)**

| **Organ** | **⁶⁸Ga-FAP-04 (% ID/g)** | | | |
|---|---|---|---|---|
| | **0.5 h** | **1 h** | **2 h** | **4 h** |
| Tumour (max) | 13.17 + 3.28 | 10.98 ± 2.02 | 10.38 ± 2.02 | 12.40 ± 0.85 |
| Tumour (mean) | 7.38 ± 1.41 | 6.39 + 0.92 | 5.82 ± 1.01 | 4.51 ± 0.28 |
| Kidney | 2.20 ± 0.10 | 1.87 ± 0.20 | 1.70 ± 0.17 | 1.35 ± 0.23 |
| Liver | 1.54 ± 0.23 | 1.11 ± 0.31 | 1.02 ± 0.23 | 1.01 ± 0.29 |
| Brain | 1.16 ± 0.28 | 0.70 + 0.09 | 0.65 + 0.21 | 0.55 ± 0.17 |
| Lung | 1.56 ± 0.45 | 1.10 ± 0.12 | 1.02 + 0.21 | 0.83 ± 0.20 |
| Muscle | 0.48 ± 0.39 | 0.46 ± 0.10 | 0.57 ± 0.27 | 0.42 ± 0.10 |
| Shoulder joint | 2.82 ± 0.57 | 2.35 ± 0.44 | 2.10 ± 0.36 | 1.74 ± 0.42 |
| Gallbladder | 1.51 ± 0.18 | 1.17 ± 0.36 | 1.09 ± 0.36 | 0.95 + 0.46 |

**Table 3.2 Uptake of the compounds of the present disclosure in tumour (tissues)**

| **Organ** | **Compound 5 (% ID/g)** | | | |
|---|---|---|---|---|
| | **0.5 h** | **1 h** | **2 h** | **4 h** |
| Tumour (max) | 12.10 ± 2.61 | 11.78 + 1.74 | 11.90 + 1.70 | 12.18 + 2.21 |
| Tumour (mean) | 8.19 ± 2.16 | 7.09 ± 1.22 | 5.55 ± 2.08 | 4.70 ± 0.94 |
| Kidney | 1.47 ± 0.44 | 1.01 + 0.29 | 1.00 ± 0.39 | 1.11 ± 0.30 |
| Liver | 0.97 ± 0.23 | 0.91 ± 0.32 | 0.89 + 0.28 | 0.86 ± 0.28 |
| Brain | 0.74 ± 0.09 | 0.55 ± 0.11 | 0.48 ± 0.05 | 0.42 ± 0.13 |
| Lung | 1.08 ± 0.14 | 0.93 ± 0.16 | 0.84 ± 0.19 | 0.70 ± 0.15 |
| Muscle | 0.59 ± 0.21 | 0.61 ± 0.17 | 0.42 + 0.06 | 0.32 ± 0.12 |
| Shoulder joint | 2.67 ± 0.41 | 2.00 ± 0.32 | 1.75 ± 0.32 | 1.41 ± 0.37 |
| Gallbladder | 1.23 ± 0.32 | 1.09 ± 0.41 | 0.93 ± 0.32 | 1.07 ± 0.52 |

**Table 3.3 Uptake of the compounds of the present disclosure in tumour (tissues)**

| **Organ** | **Compound 7 (% ID/g)** | | |
|---|---|---|---|
| | **0.5 h** | **1.5 h** | **3 h** |
| Tumour (max) | 17.50 ± 0.55 | 34.63 + 5.74 | 33.57 + 6.24 |
| Tumour (mean) | 12.09 ± 0.12 | 25.31 ± 3.31 | 20.37 + 5.54 |
| Kidney | 1.93 + 1.24 | 1.94 + 0.70 | 1.75 ± 1.17 |
| Liver | 2.04 ± 0.30 | 1.34 ± 0.33 | 0.95 ± 0.54 |
| Brain | 1.76 ± 0.41 | 0.64 ± 0.03 | 0.48 ± 0.03 |
| Lung | 2.57 ± 1.73 | 0.30 ± 0.09 | 0.23 ± 0.05 |
| Muscle | 1.19 ± 0.72 | 0.83 ± 0.07 | 0.62 ± 0.19 |
| Shoulder joint | 1.18 + 1.01 | 0.82 + 0.07 | 0.65 ± 0.16 |
| Gallbladder | 2.18 + 2.68 | 4.06 ± 1.18 | 3.41 ± 0.74 |
| Intestine | 1.26 + 0.30 | 0.64 ± 0.12 | 0.45 ± 0.05 |

**Table 3.4 Ratios of the compounds of the present disclosure in tumour tissue to non-target organs (tissues)**

| **Tumour/tissue (organ)** | **⁶⁸Gₐ-FAPI-04** | | | | **Compound 5** | | | |
|---|---|---|---|---|---|---|---|---|
| | **0.5 h** | **1.0 h** | **2.0 h** | **4.0 h** | **0.5 h** | **1.0 h** | **2.0 h** | **4.0 h** |
| **Tumour/kidney** | 3.35 | 3.42 | 3.42 | 3.34 | 5.57 | 7.02 | 5.55 | 4.23 |
| **Tumour/liver** | 4.79 | 5.76 | 5.71 | 4.47 | 8.44 | 7.79 | 6.24 | 5.47 |
| **Tumour/brain** | 6.36 | 9.13 | 8.95 | 8.20 | 11.07 | 12.89 | 11.56 | 11.19 |
| **Tumour/lung** | 4.73 | 5.81 | 5.71 | 5.43 | 7.58 | 7.62 | 6.61 | 6.71 |
| **Tumour/muscle** | 15.38 | 13.89 | 10.21 | 10.74 | 13.88 | 11.62 | 13.21 | 14.69 |
| **Tumour/shoulder joint** | 2.62 | 2.72 | 2.77 | 2.59 | 3.07 | 3.55 | 3.17 | 3.33 |
| **Tumour/gallbladde r** | 4.89 | 5.46 | 5.34 | 4.75 | 6.66 | 6.50 | 5.97 | 4.39 |

**Table 3.5 Ratios of the compounds of the present disclosure in tumour tissue to non-target organs (tissues)**

| **Tumour/tissue (organ)** | **Compound 6** | | | | **Compound 7** | | |
|---|---|---|---|---|---|---|---|
| | **0.5 h** | **1.0 h** | **2.0 h** | **4.0 h** | **0.5 h** | **1.5 h** | **3.0 h** |
| **Tumour/kidney** | 2.09 | 3.09 | 2.65 | 1.28 | 6.26 | 13.05 | 11.64 |
| **Tumour/liver** | 5.06 | 6.92 | 6.18 | 4.41 | 5.93 | 18.89 | 21.44 |
| **Tumour/brain** | 7.00 | 8.96 | 9.64 | 7.46 | 6.87 | 39.55 | 42.44 |
| **Tumour/lung** | 5.93 | 8.62 | 9.27 | 7.46 | 4.70 | 84.37 | 88.56 |
| **Tumour/muscle** | 8.40 | 11.43 | 12.05 | 9.70 | 10.16 | 30.49 | 32.85 |
| **Tumour/shoulder joint** | 2.39 | 3.22 | 3.89 | 3.03 | 10.25 | 30.87 | 31.34 |
| **Tumour/gallbladde r** | 2.63 | 2.36 | 1.43 | 0.73 | 5.55 | 6.23 | 5.97 |
| **Tumour/intestine** | / | / | / | / | 9.60 | 39.55 | 45.27 |

Conclusions: Compared with FAPI-04, compound 5 exhibits higher uptake in tumours, lower uptake in non-target organs (tissues), and superior tumour-to-non-target organ (tissue) ratios. Compared with FAPI-04, compound 7 exhibits higher uptake in tumours and superior tumour/non-target organ (tissue) ratios.

### Comparative Example 1. PET imaging test of ¹⁸F-FAPI-42

Using the same experimental method as in Example 2, the ratio of radioactive accumulation of the labelled compound ¹⁸F-FAPI-42 in tumours to non-target tissues in tumour-bearing mice was observed.

**Table 4.1 Ratios of ¹⁸F-FAPI-42 in tumours to non-target organs (tissues)**

| **Tumour/tissue (organ)** | 0.5 h | 1.5 h | 3.0 h |
|---|---|---|---|
| **Tumour/gallbladder** | 1.21 | 1.42 | 3.15 |
| **Tumour/intestine** | 3.14 | 1.28 | 1.19 |

Experimental conclusions: In the U-87 mouse model, compound 7 exhibits lower uptake in non-target organs such as the gallbladder and intestine than ¹⁸F-FAPI-42, indicating that compound 7 has higher safety. Moreover, compound 7 exhibits higher tumour/gallbladder and tumour/intestine uptake ratios, indicating that compound 7 can better distinguish tumour lesions from normal tissue and organs and has excellent targeting properties.

## Claims

1. A compound represented by formula (I) or a pharmaceutically acceptable salt thereof, 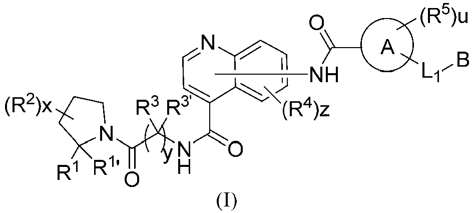
**characterized in that** R¹ and R¹' are each independently selected from hydrogen, cyano, carboxyl, sulfo, phosphono or B(OH)₂;
each R² is independently selected from hydroxyl, halogen, C₁₋₆ alkyl, halo-C₁₋₆ alkyl, -NR'R", -O-C₁₋₆ alkyl or -S-C₁₋₆ alkyl;
R³ and R³' are each independently selected from hydrogen, hydroxyl, halogen or C₁₋₆ alkyl;
R⁴ is selected from hydroxyl, halogen, C₁₋₆ alkyl, halo-C₁₋₆ alkyl, -NR'R", -O-C₁₋₆ alkyl or -S-C₁₋₆ alkyl;
ring A is selected from 3- to 12-membered cycloalkylene or 3- to 12-membered heterocycloalkylene;
R⁵ is selected from halogen, hydroxyl, C₁₋₆ alkyl, halo-C₁₋₆ alkyl, -NR'R" or - O-C₁₋₆ alkyl;
L₁ is selected from a linking bond, -NH-, -CH₂-NH- or -CH₂CH₂-NH-;
x is selected from 0, 1, 2 or 3;
R' and R" are each independently selected from hydrogen, C₁₋₆ alkyl or halo-C₁₋₆ alkyl;
y is selected from 1 or 2;
z is selected from 0, 1, 2 or 3;
u is selected from 0, 1 or 2; and
B is selected from any optically- or radio-labelled functional group suitable for optical imaging, positron emission tomography, single-photon emission computed tomography or radiation therapy; preferably, B is composed of a chelating agent and a radioactive element.

2. The compound or the pharmaceutically acceptable salt thereof according to claim 1, **characterized in that** ring A is 4- to 7-membered cycloalkylene, preferably cyclohexylene.

3. The compound or the pharmaceutically acceptable salt thereof according to claim 1 or 2, **characterized in that** R¹ and R^{1'} are each independently selected from hydrogen or cyano.

4. The compound or the pharmaceutically acceptable salt thereof according to any one of claims 1 to 3, **characterized in that** each R² is independently selected from hydroxyl, halogen or C₁₋₆ alkyl, and x is selected from 2 or 3; preferably, each R² is independently selected from fluorine or chlorine.

5. The compound or the pharmaceutically acceptable salt thereof according to any one of claims 1 to 4, **characterized in that** R³ and R³' are each independently selected from hydrogen, halogen or C₁₋₆ alkyl; preferably, R³ and R³' are both hydrogen.

6. The compound or the pharmaceutically acceptable salt thereof according to any one of claims 1 and 3 to 5, **characterized by** being a compound represented by formula (II-1) or a pharmaceutically acceptable salt thereof, 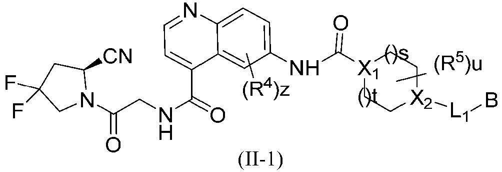
wherein X₁ and X₂ are each independently selected from CH or N,
s and t are each independently selected from 0, 1 or 2, and
R⁴, R⁵, L₁, B, z and u are as defined in claim 1.

7. The compound or the pharmaceutically acceptable salt thereof according to claim 6, **characterized in that**
i) X₁ is CH, and X₂ is N; alternatively
ii) X₁ is N, and X₂ is CH; alternatively
iii) X₁ is CH, and X₂ is CH;
preferably, X₁ is CH, and X₂ is CH.

8. The compound or the pharmaceutically acceptable salt thereof according to claim 6 or 7, **characterized in that** s and t are each independently selected from 0 or 1; preferably, s and t are both 1.

9. The compound or the pharmaceutically acceptable salt thereof according to any one of claims 6 to 8, **characterized in that** L₁- is CH₂-NH-.

10. The compound or the pharmaceutically acceptable salt thereof according to any one of claims 6 to 9, **characterized in that** X₁ is CH, and X₂ is CH; s and t are both 1; L₁ is selected from CH₂-NH- or -CH₂CH₂-NH-, preferably -CH₂-NH-.

11. The compound or the pharmaceutically acceptable salt thereof according to any one of claims 1 and 3 to 5, **characterized by** being a compound represented by formula (II-2) or a pharmaceutically acceptable salt thereof, 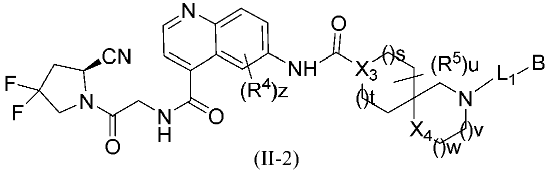
wherein X₃ and X₄ are each independently selected from CH, N or O;
s and t are each independently selected from 0, 1 or 2;
w and v are each independently selected from 0, 1 or 2; and
R⁴, R⁵, L₁, B, z and u are as defined in claim 1.

12. The compound or the pharmaceutically acceptable salt thereof according to claim 11, **characterized in that**
X₃ is CH, and X₄ is O; alternatively
X₃ is CH, and X₄ is N, or
X₃ is N, and X₄ is CH;
preferably, X₃ is CH, and X₄ is O.

13. The compound or the pharmaceutically acceptable salt thereof according to claim 11 or 12, **characterized in that** s and t are each independently selected from 0 or 1, and w and v are each independently selected from 0 or 1;
preferably, s and t are both 1, and w and v are both 1.

14. The compound or the pharmaceutically acceptable salt thereof according to any one of claims 11 to 13, **characterized in that** L₁ is a linking bond.

15. The compound or the pharmaceutically acceptable salt thereof according to any one of claims 11 to 14, **characterized in that** X₃ is CH, and X₄ is O; s and t are both 1, and w and v are both 1; L₁ is a linking bond.

16. The compound or the pharmaceutically acceptable salt thereof according to any one of claims 1 to 15, **characterized in that** R⁴ is selected from hydroxyl, halogen or C₁₋₆ alkyl, and z is selected from 0, 1, 2 or 3;
preferably, R⁴ is selected from fluorine, chlorine, methyl or ethyl, and z is selected from 0 or 1.

17. The compound or the pharmaceutically acceptable salt thereof according to any one of claims 1 to 14, **characterized in that** R⁵ is selected from hydroxyl, halogen or C₁₋₆ alkyl, and u is selected from 0, 1 or 2;
preferably, R⁵ is selected from fluorine, chlorine, methyl or ethyl, and u is selected from 0 or 1.

18. The compound or the pharmaceutically acceptable salt thereof according to any one of claims 1 to 15, **characterized in that** the chelating agent in the B is selected from: 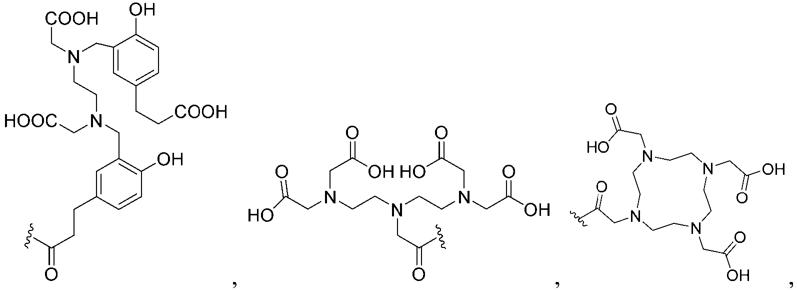 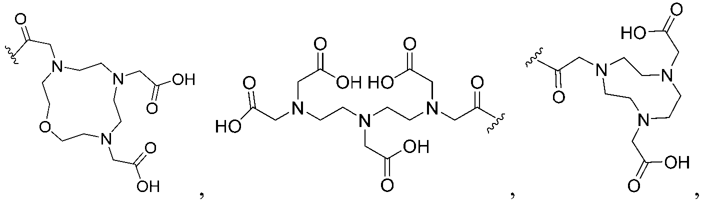 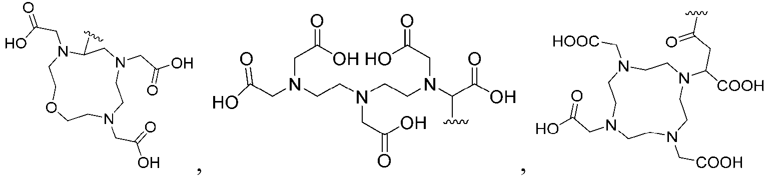 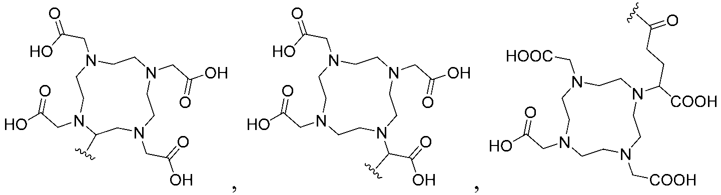 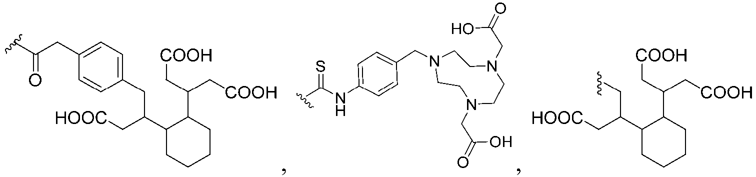 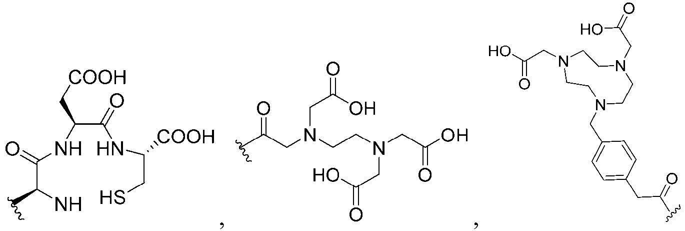 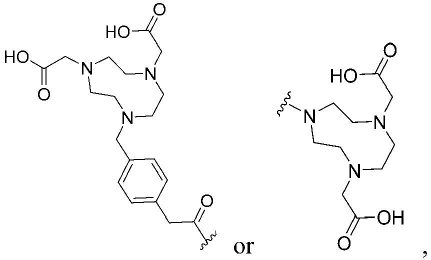 preferably 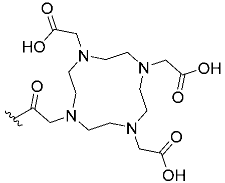 or

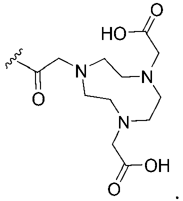

19. The compound or the pharmaceutically acceptable salt thereof according to any one of claims 1 to 18, **characterized in that** the B comprises a radioactive element, and the radioactive element is selected from ²²³Ra, ⁸⁹Sr, ^{94m}Tc, ^{99m}Tc, ¹⁸⁶Re, ¹⁸⁸Re, ²⁰³Pb, ²¹²Pb, ⁶⁷Ga, ⁶⁸Ga, ⁴⁷Sc, ¹¹¹In, ⁹⁷Ru, ⁶²Cu, ⁶⁴Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ¹²³I, ¹²⁴I, ¹²⁵I_{,} ¹⁸F, ²¹¹At, ²²⁵Ac, ⁸⁹Sr, ^{117m}Sn or ¹⁶⁹Er, preferably ¹⁸F or ⁶⁸Ga;
alternatively, the radioactive element is selected from: ⁴³Sc, ⁴⁴Sc, ⁵¹Mn, ⁵²Mn, ¹⁵²Tb, ¹⁵⁵Tb, ²⁰¹TI, ⁷⁶Br, ⁷⁷Br, ⁸⁹Zr, ⁴⁷Sc, ⁶⁷Cu, ¹⁴⁹Tb, ²¹³Bi, ²²⁶Th, ²²⁷Th or ¹³¹I.

20. The compound or the pharmaceutically acceptable salt thereof according to any one of claims 1 to 19, **characterized by** being selected from 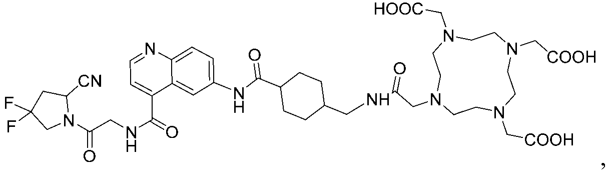 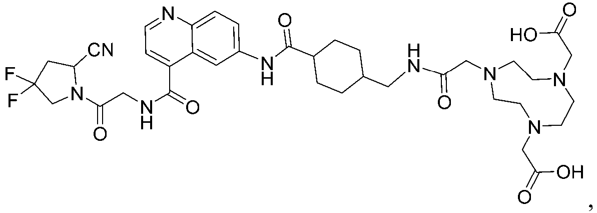 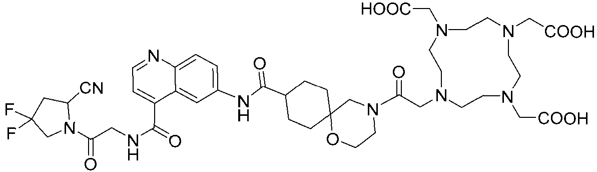 or 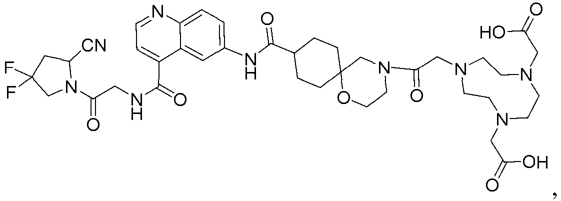 preferably 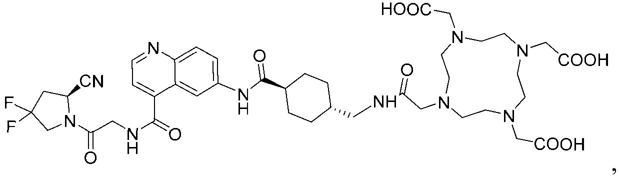 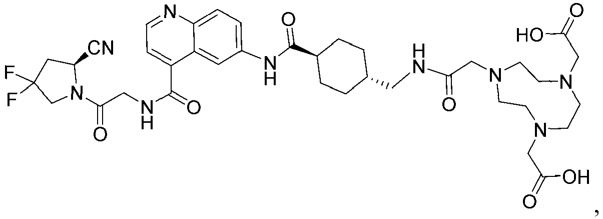 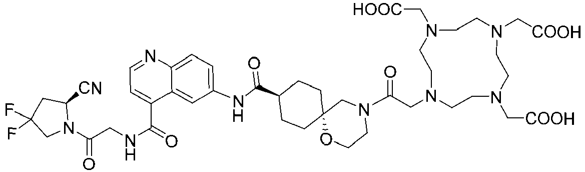 or 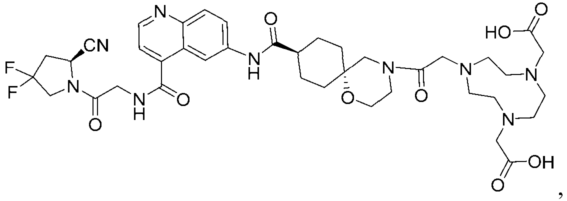 particularly selected from

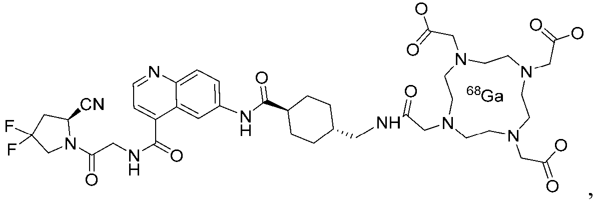

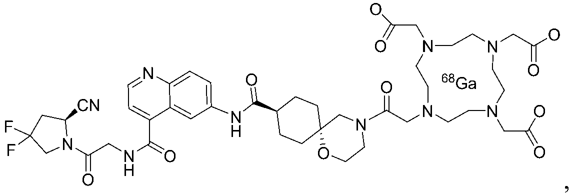

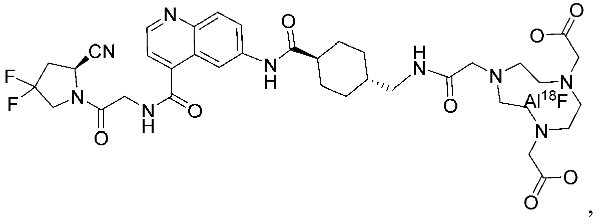

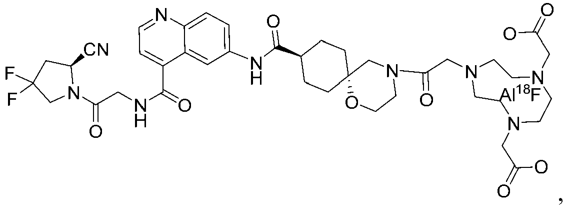

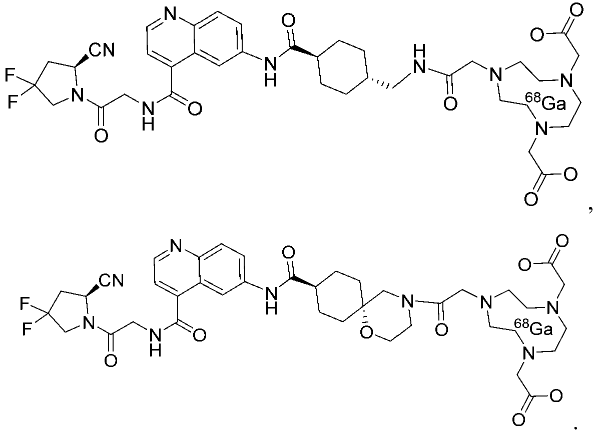

21. A pharmaceutical composition comprising the compound or the pharmaceutically acceptable salt thereof according to any one of claims 1 to 20, and one or more pharmaceutically acceptable carriers, diluents or excipients.

22. A method for preparing the compound or the pharmaceutically acceptable salt thereof according to claim 20, or the pharmaceutical composition according to claim 21, **characterized by** comprising a step of complexing the compound or the pharmaceutically acceptable salt thereof with a radioactive element.

23. Use of the compound or the pharmaceutically acceptable salt thereof according to any one of claims 1 to 20, or the pharmaceutical composition according to claim 21 in the preparation of a medicament for imaging a disease or disorder related to fibroblast activation protein, or for treating a disease or disorder related to fibroblast activation protein.

24. The use according to claim 23, **characterized in that** the disease or disorder related to fibroblast activation protein is selected from proliferative diseases, chronic inflammation, fibrosis (liver, kidney, lung), tissue remodelling, scarring disorders, tissue infections or inflammatory lesions, the proliferative diseases are selected from the group consisting of breast cancer, colorectal cancer, ovarian cancer, prostate cancer, pancreatic cancer, thyroid cancer, lung adenocarcinoma, kidney cancer, liver cancer, lung cancer, oesophageal cancer, hepatobiliary tract cancer, gastric cancer, nasopharyngeal cancer, head and neck cancer, bladder cancer, glioblastoma, peritoneal metastatic cancer, melanoma, fibrosarcoma, bone and connective tissue sarcoma, renal cell carcinoma, giant cell carcinoma, squamous cell carcinoma and adenocarcinoma, and benign tumours; the chronic inflammation is selected from rheumatoid arthritis, osteoarthritis, Crohn's disease or atherosclerotic plaques; the tissue remodelling occurs after myocardial infarction; the scarring disorders are selected from scar formation, scar tumours or scar keloids.

25. Use of the compound or the pharmaceutically acceptable salt thereof according to any one of claims 1 to 20, or the pharmaceutical composition according to claim 21 in the preparation of a medicament for preventing, diagnosing, or treating proliferative diseases, chronic inflammation, fibrosis (liver, kidney, lung), tissue remodelling, scarring disorders, tissue infections or inflammatory lesions, **characterized in that** the proliferative diseases are selected from the group consisting of breast cancer, colorectal cancer, ovarian cancer, prostate cancer, pancreatic cancer, thyroid cancer, lung adenocarcinoma, kidney cancer, liver cancer, lung cancer, oesophageal cancer, hepatobiliary tract cancer, gastric cancer, nasopharyngeal cancer, head and neck cancer, bladder cancer, glioblastoma, peritoneal metastatic cancer, melanoma, fibrosarcoma, bone and connective tissue sarcoma, renal cell carcinoma, giant cell carcinoma, squamous cell carcinoma and adenocarcinoma, and benign tumours; the chronic inflammation is selected from rheumatoid arthritis, osteoarthritis, Crohn's disease or atherosclerotic plaques; the tissue remodelling occurs after myocardial infarction; the scarring disorders are selected from scar formation, scar tumours or scar keloids.

26. A method for preparing compound 7 as shown below, 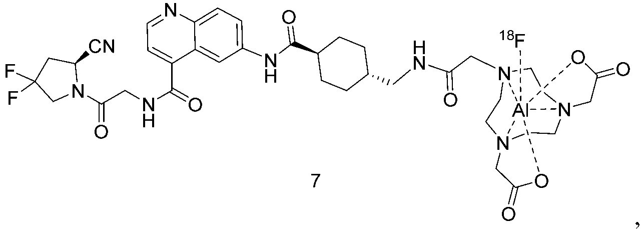 **characterized by** comprising a step of subjecting compound 2 to a reaction with ¹⁸F⁻ and AlCl₃ under acidic conditions,

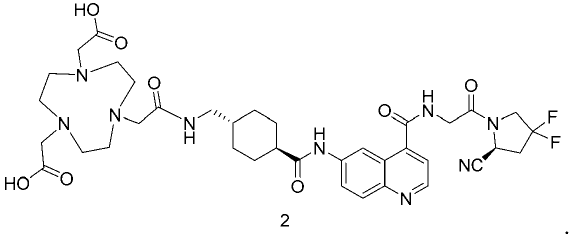

27. A method for preparing compound 2, **characterized by** comprising a step of removing a tert-butyl protecting group from compound 2-a under acidic conditions,

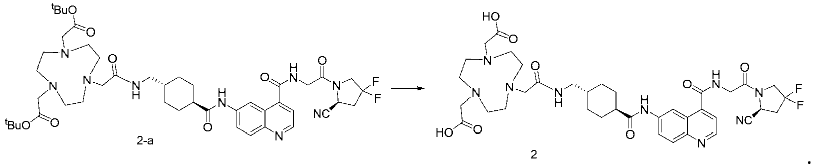

28. The method for preparing compound 7 according to claim 26, **characterized by** comprising the step of preparing compound 2 as described in claim 27.

29. The method for preparing compound 7 according to claim 26 or 28, or the method for preparing compound 2 according to claim 27, **characterized by** comprising a step of subjecting compound 1-b to a reaction with NOTA-bis(t-Bu ester) under the catalytic action of a polypeptide condensation reagent to give compound 2-a,

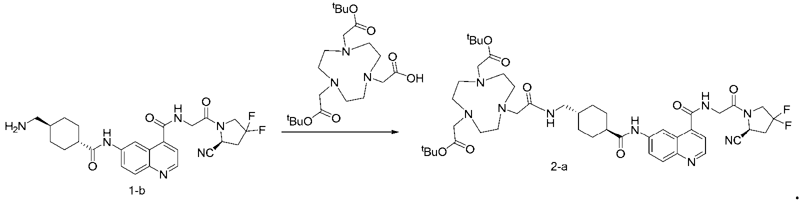

30. The method for preparing compound 7 according to any one of claims 26 and 28 to 29, or the method for preparing compound 2 according to any one of claims 27 and 29, **characterized by** comprising a step of removing a tert-butoxycarbonyl protecting group from compound 1-c under acidic or basic conditions to give compound 1-b,

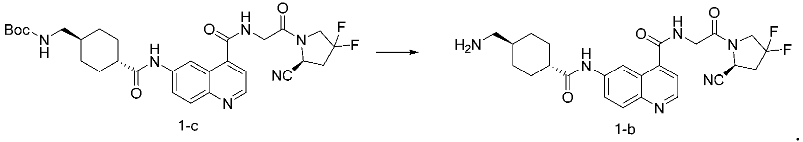

31. The method for preparing compound 7 according to any one of claims 26 and 28 to 30, or the method for preparing compound 2 according to any one of claims 27 and 29 to 30, **characterized by** comprising a step of subjecting compounds 1-f and 1-d to a reaction under the action of a condensation agent to give compound 1-c,

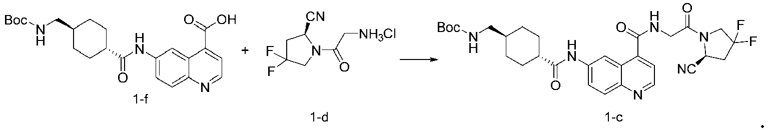

32. The method for preparing compound 7 according to any one of claims 26 and 28 to 31, or the method for preparing compound 2 according to any one of claims 27 and 29 to 31, **characterized by** comprising a step of removing a methyl protecting group from compound 1-g under basic conditions:

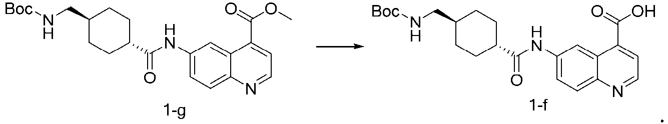

33. A compound as shown below: 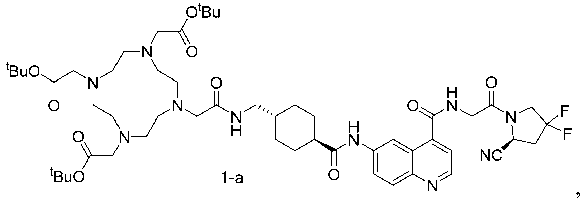 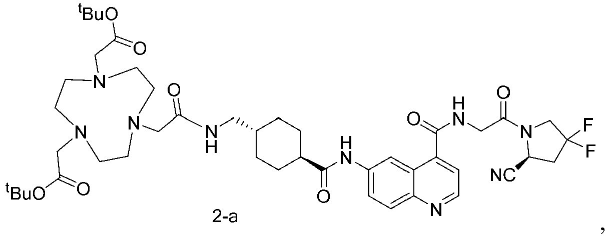 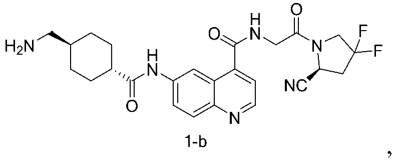 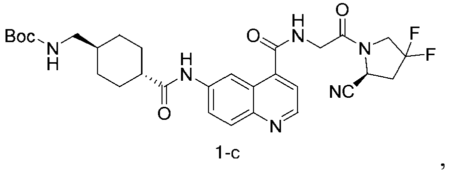 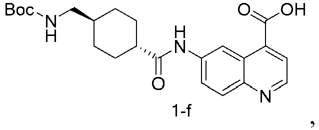 or
